(19) Europäisches Patentamt
European Patent Office
Office européen des brevets

(11) Veröffentlichungsnummer: **0 515 893 A1**

# (12) EUROPÄISCHE PATENTANMELDUNG

(21) Anmeldenummer: **92107950.5**

(22) Anmeldetag: **12.05.92**

(51) Int. Cl.5: **C07D 401/04**, A01N 43/56, C07D 403/04, C07D 405/14

(30) Priorität: **25.05.91 DE 4117076**

(43) Veröffentlichungstag der Anmeldung: **02.12.92 Patentblatt 92/49**

(84) Benannte Vertragsstaaten: **BE CH DE DK ES FR GB GR IT LI NL PT**

(71) Anmelder: **BAYER AG**

**W-5090 Leverkusen 1 Bayerwerk(DE)**

(72) Erfinder: **Fuchs, Rainer, Dr.**
**Am Rohm 107**
**W-5600 Wuppertal(DE)**
Erfinder: **Erdelen, Christoph, Dr.**
**Unterbüscherhof 22**
**W-5653 Leichlingen 3(DE)**
Erfinder: **Hartwig, Jürgen, Dr.**
**Franz-Esser-Strasse 44**
**W-5090 Leverkusen 3(DE)**
Erfinder: **Stendel, Wilhelm, Dr.**
**In den Birken 55**
**W-5600 Wuppertal(DE)**

(54) **Substituierte 4-Hetaryl-pyrazoline.**

(57) Es werden neue substituierte 4-Hetaryl-pyrazoline der allgemeinen Formel (I)

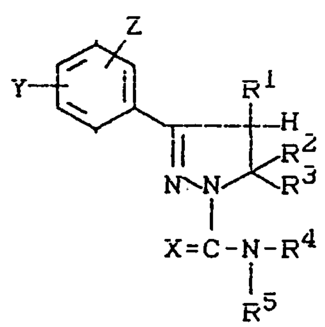

(I)

bereitgestellt, in welcher

- $R^1$ für einen ungesättigten sechsgliedrigen, 1 bis 3 Stickstoffatome enthaltenden, gegebenenfalls substituierten und gegebenenfalls benzokondensierten Heterocyclus steht,
- $R^2$ für Wasserstoff, Alkyl oder gegebenenfalls substituiertes Aryl steht,
- $R^3$ für Wasserstoff oder Alkyl steht,
- $R^4$ für Wasserstoff, Alkyl, Phenyl oder Alkylthio steht,
- $R^5$ für gegebenenfalls substituiertes Alkyl, gegebenenfalls substituiertes Cycloalkyl oder für den Rest

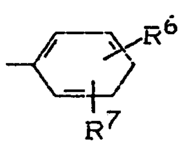

EP 0 515 893 A1

steht,
wobei $R^6$ und $R^7$ die im Anmeldungstext angegebene Bedeutung haben,

X für Sauerstoff oder Schwefel steht und

Y und Z die im Anmeldungstext angegebene Bedeutung besitzen.

Die Verbindungen (I) weisen eine stark ausgeprägte Wirksamkeit gegen tierische Schädlinge auf.

Die Erfindung betrifft neue substituierte 4-Hetarylpyrazoline, mehrere Verfahren zu ihrer Herstellung und ihre Verwendung als Schädlingsbekämpfungsmittel.

Es ist bekannt, daß bestimmte substituierte Pyrazolinderivate eine gute Wirksamkeit gegen tierische Schädlinge besitzen.

Siehe dazu z.B. DE-A 2 700 258, US-A 4 174 393, DE-A 2 529 689, US-A 4 070 365.

Die Wirkungshöhe bzw. Wirkungsdauer dieser vorbekannten Verbindungen ist jedoch, insbesondere gegen bestimmte Organismen oder bei niedrigen Anwendungskonzentrationen, nicht in allen Anwendungsgebieten völlig zufriedenstellend.

Es wurden neue substituierte 4-Hetaryl-pyrazoline der allgemeinen Formel (I)

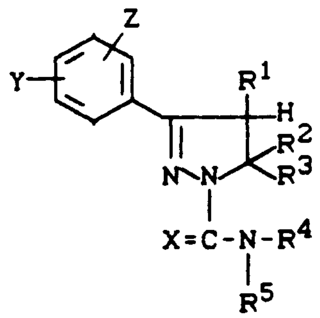

(I)

in welcher

- $R^1$ für einen ungesättigten sechsgliedrigen, 1 bis 3 Stickstoffatome enthaltenden, gegebenenfalls substituierten und gegebenenfalls benzokondensierten Heterocyclus steht,
- $R^2$ für Wasserstoff, Alkyl oder gegebenenfalls substituiertes Aryl steht,
- $R^3$ für Wasserstoff oder Alkyl steht,
- $R^4$ für Wasserstoff, Alkyl, Phenyl oder Alkylthio steht,
- $R^5$ für gegebenenfalls substituiertes Alkyl, gegebenenfalls substituiertes Cycloalkyl oder für den Rest

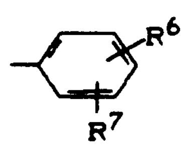

steht,

wobei $R^6$ und $R^7$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, Halogendialkylamino, ggf. substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl oder wobei $R^6$ und $R^7$ zusammen für einen bivalenten gegebenenfalls ein oder zwei Sauerstoffatome enthaltenden und gegebenenfalls substituierten Rest stehen,

- X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy, Halogenalkylthio, Alkylcarbonyl, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Amino, Nitro, Cyano oder wobei Y und Z zusammen für gegebenenfalls durch Halogen substituiertes 3,4-Methylendioxy oder 3,4-Ethylendioxy stehen,

gefunden.

Weiterhin wurde gefunden, daß man die neuen substituierten 4-Hetaryl-pyrazoline der allgemeinen Formel (I)

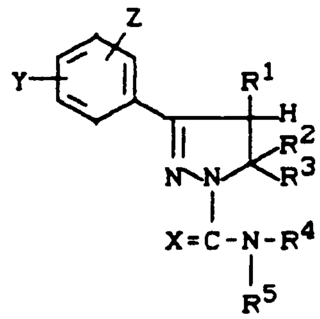

(I)

in welcher

$R^1$ für einen ungesättigten sechsgliedrigen, 1 bis 3 Stickstoffatome enthaltenden, gegebenenfalls substituierten und gegebenenfalls benzokondensierten Heterocyclus steht,

$R^2$ für Wasserstoff, Alkyl oder gegebenenfalls substituiertes Aryl steht,

$R^3$ für Wasserstoff oder Alkyl steht,

$R^4$ für Wasserstoff, Alkyl, Phenyl oder Alkylthio steht,

$R^5$ für gegebenenfalls substituiertes Alkyl, gegebenenfalls substituiertes Cycloalkyl oder für den Rest

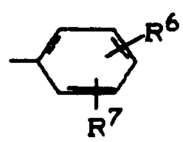

steht,

wobei $R^6$ und $R^7$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, Halogendialkylamino, ggf. substituiertes Cycloalkyl, Alkylcarbonyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl oder wobei $R^6$ und $R^7$ zusammen für einen bivalenten gegebenenfalls ein oder zwei Sauerstoffatome enthaltenden und gegebenenfalls substituierten Rest stehen,

X für Sauerstoff oder Schwefel steht und Y und Z gleich oder verschieden sein können und für Wasserstoff, Alkyl, Halogen, Halogenalkyl, Alkoxy, Alkylthio, Halogenalkoxy, Halogenalkylthio, Alkylcarbonyl, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Amino, Nitro, Cyano oder wobei Y und Z zusammen für gegebenenfalls durch Halogen substituiertes 3,4-Methylendioxy oder 3,4-Ethylendioxy stehen,

erhält, wenn man

(a) zum Erhalt von Pyrazolinderivaten der Formel (Ia), in welcher $R^4$ für Wasserstoff steht,

($\alpha$) Pyrazolinderivate der Formel (II)

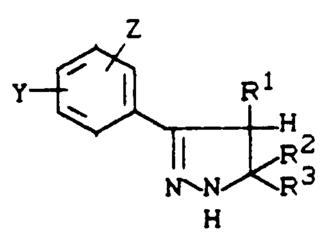

(II)

in welcher Y, Z, $R^1$, $R^2$ und $R^3$ die oben angegebene Bedeutung besitzen,
mit Isocyanaten bzw. Isothiocyanaten der Formel (III)

$X = C = N\text{-}R^5$ (III)

in welcher X und $R^5$ die oben angegebene Bedeutung haben,
gegebenenfalls in Gegenwart eines Verdünnungsmittels und gegebenenfalls in Gegenwart von Basen umsetzt

oder wenn man

($\beta$) substituierte Pyrazolcarbonsäureanilide der Formel (IV)

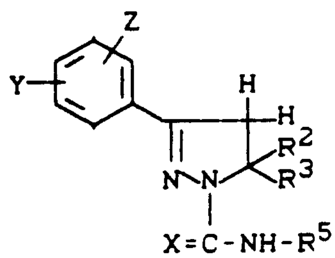

(IV)

in welcher
X, Y, Z, $R^2$, $R^3$ und $R^5$ die oben angegebene Bedeutung haben,
mit Halogeniden der Formel (V)

$Hal\text{-}R^1$ (V)

in welcher
$R^1$ die oben angegebene Bedeutung hat und
Hal für Fluor, Chlor, Brom, Iod, insbesondere Chlor, Brom oder Iod steht,
gegebenenfalls in Gegenwart eines Verdünnungsmittels und in Gegenwart einer starken Base und gegebenenfalls in Gegenwart einer Schutzgasatmosphäre umsetzt,

oder wenn man

(b) zum Erhalt von Pyrazolinderivaten der Formel (Ib), in welcher $R^4$ für Alkyl, Phenyl oder Alkylthio steht, die nach Verfahren (a-$\alpha$) und (a-$\beta$) erhältlichen Pyrazolinderivate der Formel (Ia),

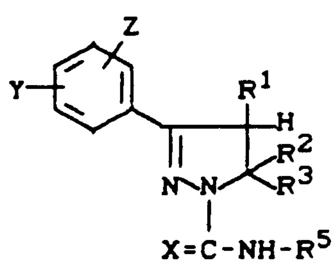

(Ia)

in welcher
X, Y, Z, $R^1$, $R^2$, $R^3$ und $R^5$ die oben angegebene Bedeutung haben,
mit Alkylierungsmitteln der Formel (VI)

$R^4\text{-}E$ (VI)

in welcher
$R^4$ für Alkyl, Phenyl oder Alkylthio steht und
E für eine elektronenanziehende Abgangsgruppe steht,
in wasserfreiem Medium unter Zusatz einer starken Base umsetzt.

Schließlich wurde gefunden, daß die neuen substituierten 4-Hetaryl-pyrazoline der allgemeinen Formel (I) eine sehr gute Wirksamkeit gegen tierische Schädlinge und insbesondere eine sehr gute insektizide und

akarizide Wirksamkeit besitzen.

Überraschenderweise zeigen die erfindungsgemäßen substituierten 4-Hetaryl-pyrazoline eine erheblich bessere Wirksamkeit gegen pflanzenschädigende und warmblüterparasitierende Insekten und Spinnentiere als aus dem Stand der Technik bekannte chemisch und wirkungsmäßig naheliegende Verbindungen.

Die erfindungsgemäßen substituierten 4-Hetaryl-pyrazoline sind durch die Formel (I) allgemein definiert. Bevorzugt sind Verbindungen der Formel (I), bei welchen

$R^1$ für einen gegebenenfalls durch Halogen, Hydroxy, Alkyl($C_1$-$C_6$), CN, $NO_2$, Alkoxy($C_1$-$C_6$)carbonyl, Alkyl($C_1$-$C_4$)thio, Alkoxy($C_1$-$C_6$), Halogenalkyl($C_1$-$C_4$), Halogenalkyl($C_1$-$C_4$)thio, Halogenalkoxy($C_1$-$C_4$), Dialkyl-($C_1$-$C_4$)amino oder Dihalogenamino substituierten Pyridin-, Pyridon-, Pyrimidin-, Pyrimidon-, Pyridazinon-, Pyrazinon-, Pyrazin-, Pyridazin- oder Triazin-Rest steht,

$R^2$ für Wasserstoff, Alkyl($C_1$-$C_6$) oder gegebenenfalls einfach bis dreifach, gleich oder verschieden substituiertes Phenyl steht, wobei als Substituenten genannt seien: Halogen, Cyano, Alkyl($C_1$-$C_6$), Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio,

$R^3$ für Wasserstoff oder Alkyl($C_1$-$C_6$) steht,

$R^4$ für Wasserstoff, Alkyl($C_1$-$C_6$), Phenyl oder Alkyl($C_1$-$C_4$)thio steht,

$R^5$ für gegebenenfalls durch Halogen, Halogenalkyl($C_1$-$C_4$), Halogenalkoxy($C_1$-$C_4$) substituiertes Alkyl($C_1$-$C_6$),

für gegebenenfalls durch Halogen, Halogenalkyl($C_1$-$C_4$),Halogenalkoxy($C_1$-$C_4$) substituiertes Cycloalkyl ($C_3$-$C_7$) oder für den Rest

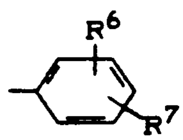

steht,

wobei $R^6$ und $R^7$ gleich oder verschieden sein können und für Wasserstoff, Halogen, Alkyl($C_1$-$C_6$), Nitro, Cyano, Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio, gegebenenfalls durch Halogen, Halogenalkyl($C_1$-$C_6$), Alkoxy($C_1$-$C_6$), Alkyl($C_1$-$C_6$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_6$)carbonyl, Alkenyl($C_2$-$C_6$)oxy, Alkinyl($C_2$-$C_6$), Alkyl-($C_1$-$C_4$)thionyl, Alkyl($C_1$-$C_4$)sulfonyl, Halogenalkyl($C_1$-$C_4$)thionyl, Halogenalkyl($C_1$-$C_4$)-sulfonyl, Halogenalkoxy($C_1$-$C_4$)-carbonyl, Alkyl($C_1$-$C_4$)-carbonyl stehen oder wobei

$R^6$ und $R^7$ zusammen für einen der folgenden bivalenten Reste stehen:

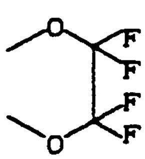 , 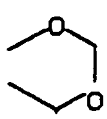 , 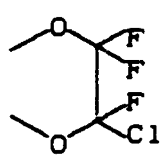 , 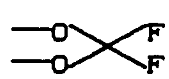 ,

X für Sauerstoff oder Schwefel steht und

Y und Z gleich oder verschieden sein können und für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_4$), Halogenalkyl($C_1$-$C_4$)-thio, Alkyl ($C_1$-$C_4$)carbonyl, Alkoxy($C_1$-$C_4$)carbonyl, Halogenalkoxy($C_1$-$C_4$)-carbonyl, gegebenenfalls durch Halogen, Alkyl($C_1$-$C_4$), Alkoxy($C_1$-$C_4$), Halogenalkyl ($C_1$-$C_4$) substituiertes Phenoxy oder Phenylthio, Alkenyl($C_2$-$C_6$)oxy, Alkinyl($C_2$-$C_6$), Alkyl($C_1$-$C_4$)-thionyl, Alkyl($C_1$-$C_4$)sulfonyl, Halogenalkyl($C_1$-$C_4$)thionyl, Halogenalkyl($C_1$-$C_4$)sulfonyl, gegebenenfalls durch Halogen, Alkoxy-($C_1$-$C_4$), Halogenalkyl($C_1$-$C_4$) substituiertes Mono- oder Dialkyl($C_1$-$C_6$)amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

Besonders bevorzugt sind Verbindungen der Formel (I), bei welchen

$R^1$ für einen gegebenenfalls durch Fluor, Chlor, Brom, Iod, Hydroxy, Alkyl($C_1$-$C_4$), CN, $NO_2$, Alkoxy-($C_1$-$C_4$)carbonyl, Alkyl($C_1$-$C_3$)thio, Alkoxy($C_1$-$C_4$), Halogenalkyl($C_1$-$C_3$), Halogenalkyl($C_1$-$C_3$)thio oder Halogenalkoxy($C_1$-$C_4$)carbonyl substituierten Pyridin-, Pyridon-, Pyrimidin-, Pyrimidon-, Pyridazinon-, Pyrazinon-, Pyrazin-, Pyridazin- oder Triazin-Rest steht,

$R^2$ für Wasserstoff, Alkyl($C_1$-$C_4$) oder gegebenenfalls durch Halogen, Cyano, Alkyl($C_1$-$C_3$), Halogenalkyl($C_1$-$C_3$), Alkoxy($C_1$-$C_3$), Halogenalkoxy($C_1$-$C_3$), Alkyl($C_1$-$C_3$)thio oder Halogenalkyl-($C_1$-$C_3$)thio substituiertes Phenyl steht,

$R^3$ für Wasserstoff oder Alkyl($C_1$-$C_4$) steht,

$R^4$ für Wasserstoff, Alkyl($C_1$-$C_4$), Phenyl oder Alkyl($C_1$-$C_3$)thio steht,

$R^5$ für gegebenenfalls durch Fluor, Chlor, Brom, Halogenalkyl($C_1$-$C_3$), Halogenalkoxy($C_1$-$C_3$)-substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cycloalkyl($C_3$-$C_6$) oder für den Rest

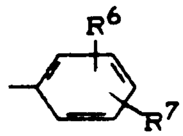

steht,

wobei $R^6$ und $R^7$ gleich oder verschieden sein können und für

Wasserstoff, Fluor, Chlor, Brom, Iod, Alkyl($C_1$-$C_4$), Nitro, Cyano, Halogenalkyl($C_1$-$C_3$), Alkoxy($C_1$-$C_4$), Halogenalkoxy ($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$), Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituierts Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$), Alkoxy-($C_1$-$C_3$)carbonyl, Alkyl($C_1$-$C_3$)thionyl, Alkyl($C_1$-$C_3$)-carbonyl, Halogenalkyl($C_1$-$C_3$)sulfuryl stehen oder wobei

$R^6$ und $R^7$ zusammen für einen der folgenden bivalenten Reste stehen;

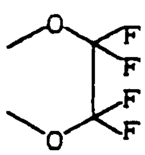 , 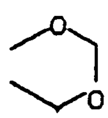 , 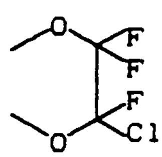 , 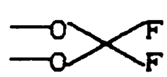 ,

X für Sauerstoff oder Schwefel steht und

Y und Z gleich oder verschieden sein können und für Wasserstoff, Alkyl($C_1$-$C_4$), Fluor, Chlor, Brom, Jod, Halogenalkyl($C_1$-$C_4$), Alkoxy($C_1$-$C_4$), Alkyl($C_1$-$C_4$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Alkyl-($C_1$-$C_3$), Alkoxy ($C_1$-$C_3$), Halogenalkyl ($C_1$-$C_3$) substituiertes Phenoxy oder Phenylthio, Halogenalkoxy($C_1$-$C_3$)carbonyl, Alkenyl($C_2$-$C_4$)oxy, Alkinyl($C_2$-$C_4$), Alkyl($C_1$-$C_3$)thionyl, Alkyl($C_1$-$C_3$)sulfonyl, Halogenalkyl($C_1$-$C_3$)thionyl, Halogenalkyl($C_1$-$C_3$)sulfonyl, gegebenenfalls durch Halogen, Alkoxy ($C_1$-$C_3$), Halogenalkyl ($C_1$-$C_3$), substituiertes Mono- oder Dialkyl($C_1$-$C_3$)amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

Ganz besonders sind bevorzugt Verbindungen der Formel (I), bei welchen

$R^1$ für einen gegebenenfalls durch Fluor, Chlor, Brom, Iod, Hydroxy, Alkyl($C_1$-$C_4$), CN, $NO_2$, Alkoxy($C_1$-$C_4$)carbonyl, Alkyl($C_1$-$C_3$)thio, Alkoxy($C_1$-$C_4$), Halogenalkyl($C_1$-$C_3$), Halogenalkyl($C_1$-$C_3$)thio, Halogenalkoxy($C_1$-$C_4$) oder Halogenalkoxy($C_1$-$C_4$)carbonyl substituierten Pyridin-, Pyridon-, Pyrimidin-, Pyrimidon-, Pyridazinon-, Pyrazinon-, Pyrazin- oder Pyridazinrest steht,

| | |
|---|---|
| $R^2$ | für Wasserstoff oder Alkyl($C_1$-$C_4$) steht, |
| $R^3$ | für Wasserstoff oder Alkyl($C_1$-$C_4$) steht, |
| $R^4$ | für Wasserstoff oder Alkyl($C_1$-$C_4$) steht und |
| $R^5$, X und Z | die oben angegebene Bedeutung haben. |

Die Substituentenbedeutung Halogenalkyl in den Resten Halogenalkyl, Halogenalkoxy, Halogenalkylthio, Halogenalkylthionyl und Halogenalkylsulfonyl enthält vorzugsweise 1 bis 4, insbesondere 1 oder 2 Kohlenstoffatome und vorzugsweise 1 bis 5, insbesondere 1 bis 3 gleiche oder verschiedene Halogenatome, wobei als Halogenatome vorzugsweise Fluor, Chlor und Brom, insbesondere Fluor und Chlor stehen. Beispielhaft seien Trifluormethyl, Chlordifluormethyl, Brommethyl, 2,2,2-Trifluorethyl und Pentafluorethyl genannt.

Die Substituentenbedeutung $R^1$ = gegebenenfalls substituierter Pyridinrest steht vorzugsweise für gegebenenfalls substituiertes Pyrid-2-yl, Pyrid-3-yl und Pyrid-4-yl.

$R^1$ = gegebenenfalls substituierter Pyridonrest steht vorzugsweise für gegebenenfalls substituiertes Pyrid-2-on-1-yl, Pyrid-3-on-1-yl und Pyrid-4-on-1-yl.

$R^1$ = gegebenenfalls substituierter Pyrimidinrest steht vorzugsweise für gegebenenfalls substituiertes Pyrimid2-yl, Pyrimid-4-yl, Pyrmid-5-yl, Pyrimidon-2-1-yl, Pyrimidon-4-3-yl.

$R^1$ = gegebenenfalls substituierter Pyrazinrest steht vorzugsweise für gegebenenfalls substituiertes Pyrazin2-yl.

$R^1$ = gegebenenfalls substituierter Pyridazinylrest steht vorzugsweise für gegebenenfalls substituiertes Pyridazin-3-yl und Pyridazin-4-yl.

Im einzelnen seien beispielhaft, aber nicht einschränkend außer den bei den Herstellungsbeispielen genannten Verbindungen die folgenden in Tabelle 1 aufgeführten, substituierten 4-Hetaryl-pyrazoline der allgemeinen Formel (I) genannt:

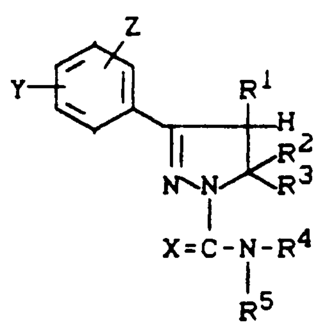

(I)

Tabelle 1:

| $R^1$ | $R^2$ | $R^3$ | $R^4$ | $R^5$ | X | Y | Z |
|---|---|---|---|---|---|---|---|
| 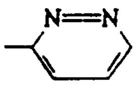 | H | H | H | 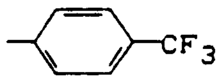 | O | H | H |
| 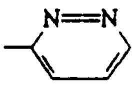 | H | H | H | 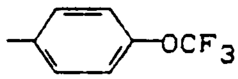 | O | H | H |
| 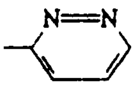 | H | H | H | 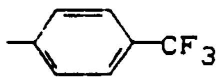 | O | 4-$CF_3$ | H |
| 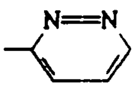 | H | H | H | 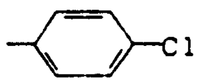 | O | 4-$CF_3$ | H |
| 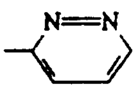 | H | H | H | 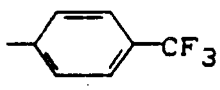 | O | 4-Br | H |

Tabelle 1 - Fortsetzung:

| $R^1$ | $R^2$ | $R^3$ | $R^4$ | $R^5$ | X | Y | Z |
|---|---|---|---|---|---|---|---|
| 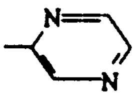 | H | H | H | 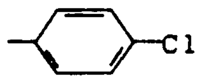 | O | 4-F | H |
| 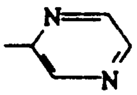 | H | H | H | 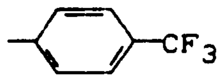 | O | 4-F | H |
| 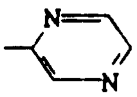 | H | H | H | 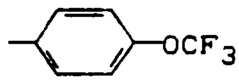 | O | 4-F | H |
| 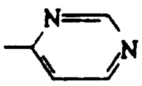 | H | H | H | 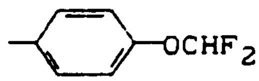 | O | 4-F | H |
| 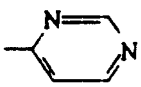 | H | H | H | 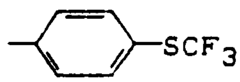 | O | 4-F | H |
| 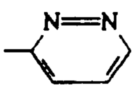 | H | H | H | 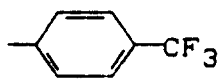 | O | 4-F | H |
| 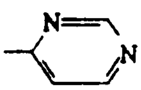 | H | H | H | 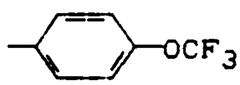 | O | 4-$OCHF_2$ | H |
| 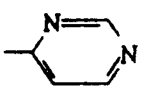 | H | H | H | 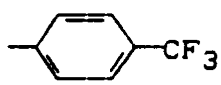 | O | 4-Cl | H |
| 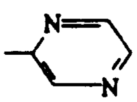 | H | H | H | 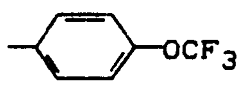 | O | 4-Cl | H |
| 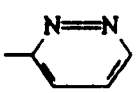 | H | H | H | 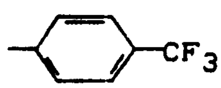 | O | 4-$OCHF_2$ | H |

Tabelle 1 - Fortsetzung:

| $R^1$ | $R^2$ | $R^3$ | $R^4$ | $R^5$ | X | Y | Z |
|---|---|---|---|---|---|---|---|
| 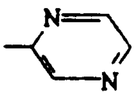 | H | H | H | 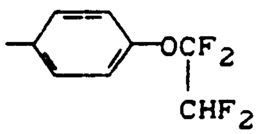 | O | 4-$OCHF_2$ | H |
| 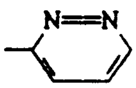 | H | H | H | 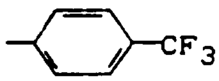 | O | 4-$CH_3$ | H |
| 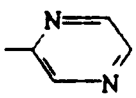 | H | H | H | 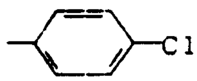 | O | 4-$CH_3$ | H |
| 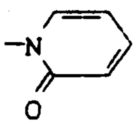 | H | H | H | 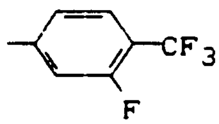 | O | H | 4-$OCHF_2$ |
| 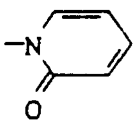 | H | H | H | 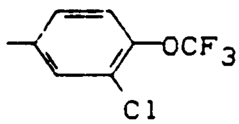 | O | H | 4-$OCHF_2$ |
| 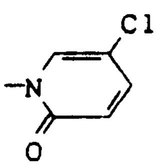 | H | H | H | 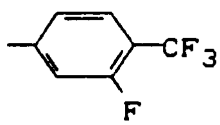 | O | H | 4-F |
| 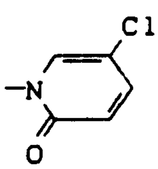 | H | H | H | 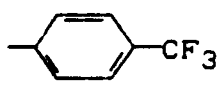 | O | H | 4-Cl |
| 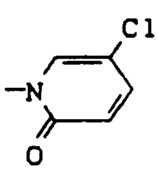 | H | H | H | 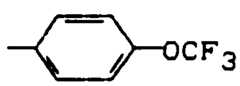 | O | H | 4-Cl |

<u>Tabelle 1</u> - Fortsetzung:

| $R^1$ | $R^2$ | $R^3$ | $R^4$ | $R^5$ | X | Y | Z |
|---|---|---|---|---|---|---|---|
| 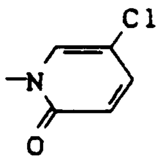 | H | H | H | 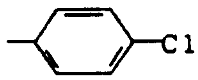 | O | H | 4-Cl |
| 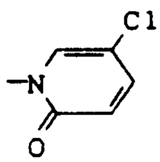 | H | H | H | 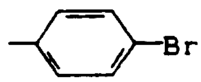 | O | H | 4-Cl |
| 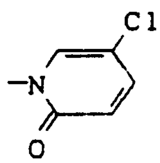 | H | H | H | 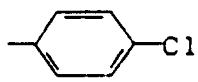 | O | H | 4-OCHF$_2$ |
| 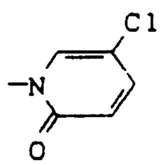 | H | H | H | 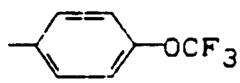 | O | H | 4-OCHF$_2$ |
| 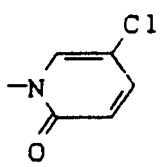 | H | H | H | 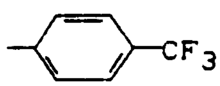 | O | H | 4-OCHF$_2$ |
| 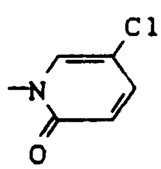 | H | H | H | 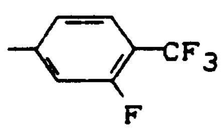 | O | H | H |
| 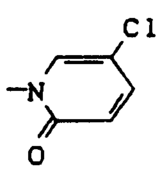 | H | H | H | 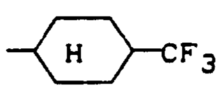 | O | H | 4-Cl |

Tabelle 1 - Fortsetzung:

| $R^1$ | $R^2$ | $R^3$ | $R^4$ | $R^5$ | X | Y | Z |
|---|---|---|---|---|---|---|---|
| 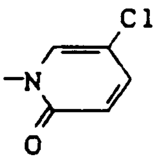 | H | H | H | 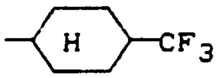 | O | H | 4-$OCHF_2$ |
| 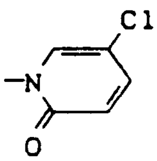 | H | H | H | 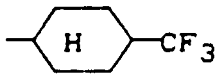 | O | H | H |
| 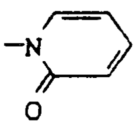 | H | H | H | 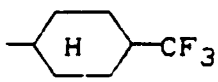 | O | H | 4-$OCHF_2$ |
| 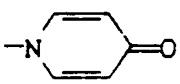 | H | H | H | 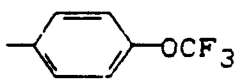 | O | H | 4-F |
| 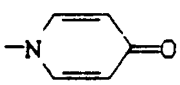 | H | H | H | 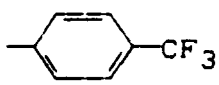 | O | H | 4-F |
| 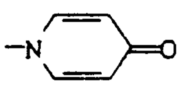 | H | H | H | 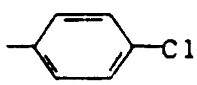 | O | H | 4-F |
| 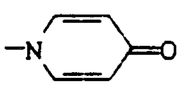 | H | H | H | 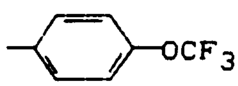 | O | H | 4-Cl |
| 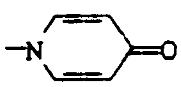 | H | H | H | 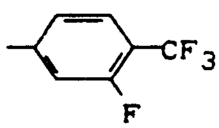 | O | H | 4-Cl |
| 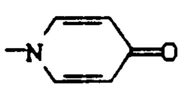 | H | H | H | 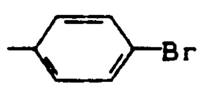 | O | H | 4-Br |

Tabelle 1 - Fortsetzung:

| $R^1$ | $R^2$ | $R^3$ | $R^4$ | $R^5$ | X | Y | Z |
|---|---|---|---|---|---|---|---|
| 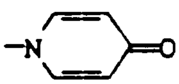 | H | H | H | 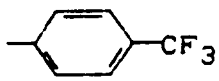 | O | H | $4\text{-}OCHF_2$ |
| 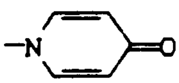 | H | H | H | 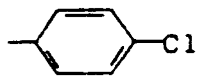 | O | H | $4\text{-}OCHF_2$ |
| 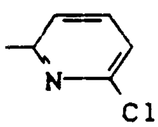 | H | H | H | 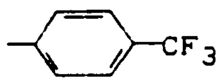 | O | H | $4\text{-}OC_2H_5$ |
| 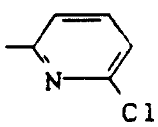 | H | H | H | 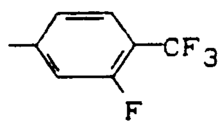 | O | H | $4\text{-}OC_2H_5$ |
| 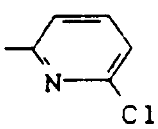 | H | H | H | 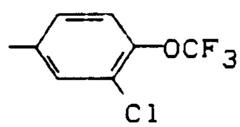 | O | H | $4\text{-}C_2H_5$ |
| 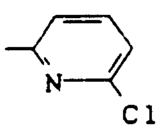 | H | H | H | 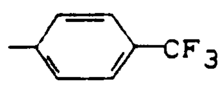 | O | $3,4\text{-}O\text{-}CF_2\text{-}O\text{-}$ | |
| 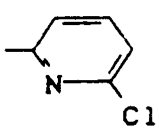 | H | H | H | 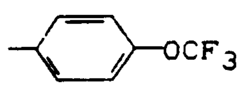 | O | $3,4\text{-}O\text{-}CF_2\text{-}O\text{-}$ | |
| 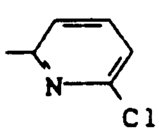 | H | H | H | 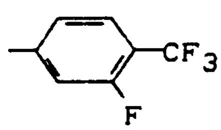 | O | $3,4\text{-}O\text{-}CF_2\text{-}O\text{-}$ | |
| 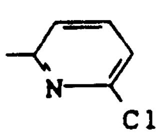 | H | H | H | 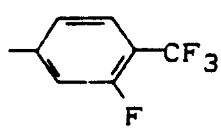 | O | H | 4-F |

Tabelle 1 - Fortsetzung:

| $R^1$ | $R^2$ | $R^3$ | $R^4$ | $R^5$ | X | Y | Z |
|---|---|---|---|---|---|---|---|
| 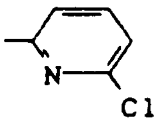 | H | H | H | 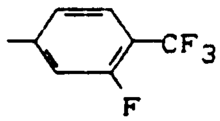 | O | H | 4-Br |
| 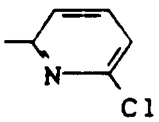 | H | H | H | 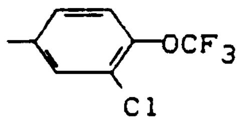 | O | H | 4-Br |
| 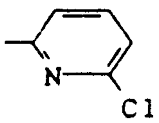 | H | H | H | 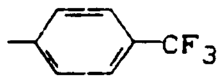 | O | H | 4-Cl |
| 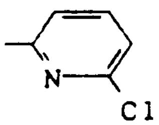 | H | H | H | 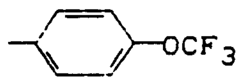 | O | H | 4-Cl |
| 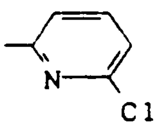 | H | H | H | 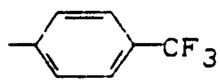 | O | H | 4-$OCHF_2$ |
| 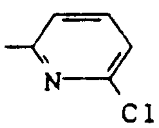 | H | H | H | 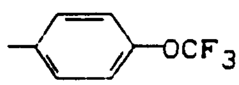 | O | H | 4-$OCHF_2$ |
| 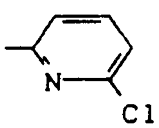 | H | H | H | 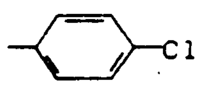 | O | H | 4-$OCHF_2$ |
| 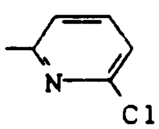 | H | H | H | 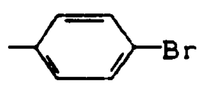 | O | H | 4-$OCHF_2$ |

<u>Tabelle 1</u> - Fortsetzung:

| $R^1$ | $R^2$ | $R^3$ | $R^4$ | $R^5$ | X | Y | Z |
|---|---|---|---|---|---|---|---|
| 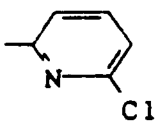 | H | H | H | 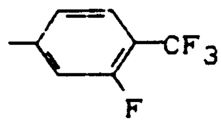 | O | H | 4-$OCF_2$-CHFCl |
| 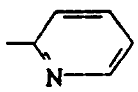 | H | H | H | 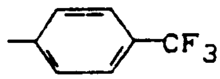 | O | 3,4-O-$CF_2$-O- | |
| 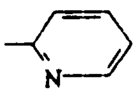 | H | H | H | 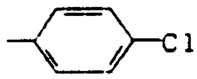 | O | 3,4-O-$CF_2$-O- | |
| 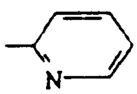 | H | H | H | 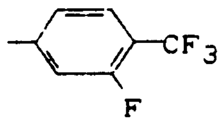 | O | 3,4-O-$CF_2$-O- | |
| 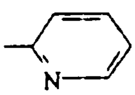 | H | H | H | 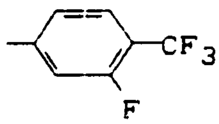 | O | H | 4-$OCHF_2$ |
| 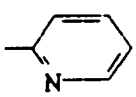 | H | H | H | 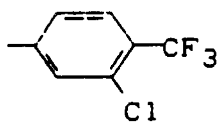 | O | H | 4-$OCHF_2$ |
| 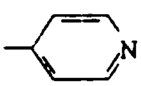 | H | H | H | 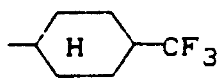 | O | H | 4-Cl |
| 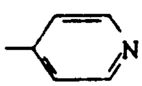 | H | H | H | 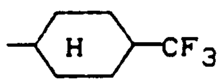 | O | H | 4-Br |
| 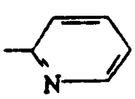 | H | H | H | 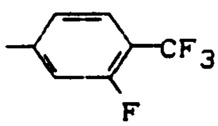 | O | H | 4-Cl |

Tabelle 1 - Fortsetzung:

| $R^1$ | $R^2$ | $R^3$ | $R^4$ | $R^5$ | X | Y | Z |
|---|---|---|---|---|---|---|---|
| 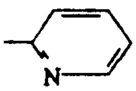 | H | H | H | 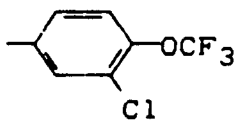 | O | H | 4-Cl |
| 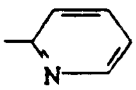 | H | H | H | 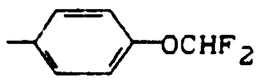 | O | H | 4-Cl |
| 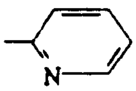 | H | H | H | 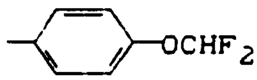 | O | H | 4-F |
| 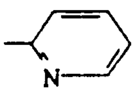 | H | H | H | 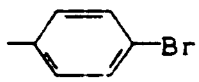 | O | H | 4-Cl |
| 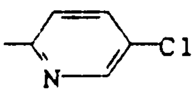 | H | H | H | 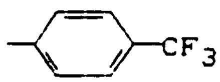 | O | H | 4-Cl |
| 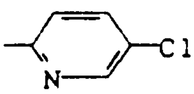 | H | H | H | 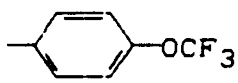 | O | H | 4-F |
| 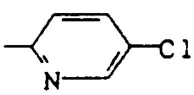 | H | H | H | 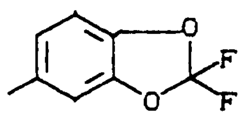 | O | H | 4-Cl |
| 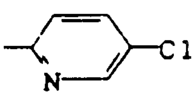 | H | H | H | 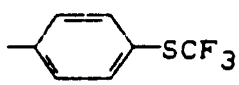 | O | H | 4-Cl |
| 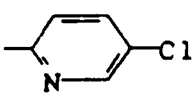 | H | H | H | 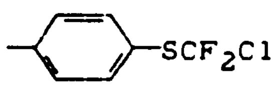 | O | H | 4-F |
| 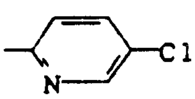 | H | H | H | 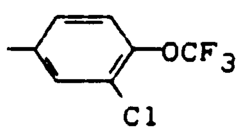 | O | H | 4-Cl |

Tabelle 1 - Fortsetzung:

| $R^1$ | $R^2$ | $R^3$ | $R^4$ | $R^5$ | X | Y | Z |
|---|---|---|---|---|---|---|---|
| 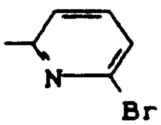 | H | H | H | 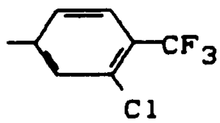 | O | H | 4-F |
| 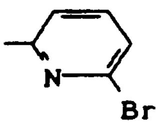 | H | H | H | 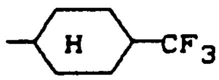 | O | H | 4-Cl |
| 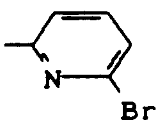 | H | H | H | 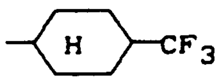 | O | H | 4-F |
| 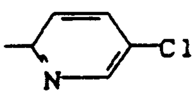 | H | H | H | 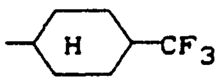 | O | H | 4-Cl |
| 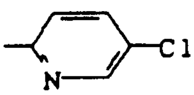 | H | H | H | 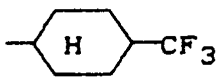 | O | H | 4-F |
| 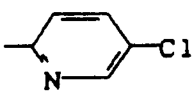 | H | H | H | 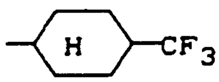 | O | H | 4-$OC_2H_5$ |
| 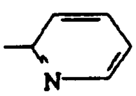 | H | H | H | 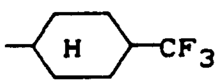 | O | H | H |
| 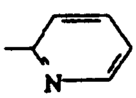 | H | H | H | 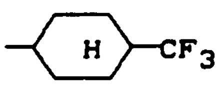 | O | H | 4-$OC_2H_5$ |
| 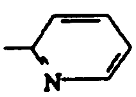 | -$C_2H_5$ | H | H | 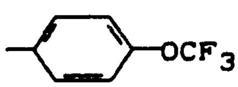 | O | H | 4-Cl |

Tabelle 1 - Fortsetzung:

| $R^1$ | $R^2$ | $R^3$ | $R^4$ | $R^5$ | X | Y | Z |
|---|---|---|---|---|---|---|---|
| 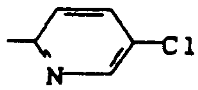 | H | 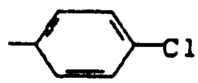 | H | 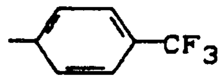 | O | H | 4-F |
| 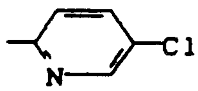 | H | H | $-CH_3$ | 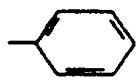 | O | H | 4-Cl |
| 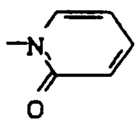 | $-CH_3$ | $-CH_3$ | H | 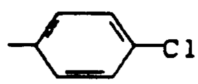 | O | H | 4-Cl |

Verwendet man beispielsweise 3-(4-Fluorphenyl)-4-pyrid-2-on-1-yl-4,5-dihydropyrazol und 4-Trifluormethylphenylisocyanat als Ausgangsstoffe, so läßt sich der Reaktionsablauf des erfindungsgemäßen Verfahrens (a-α) durch das folgende Formelschema darstellen:

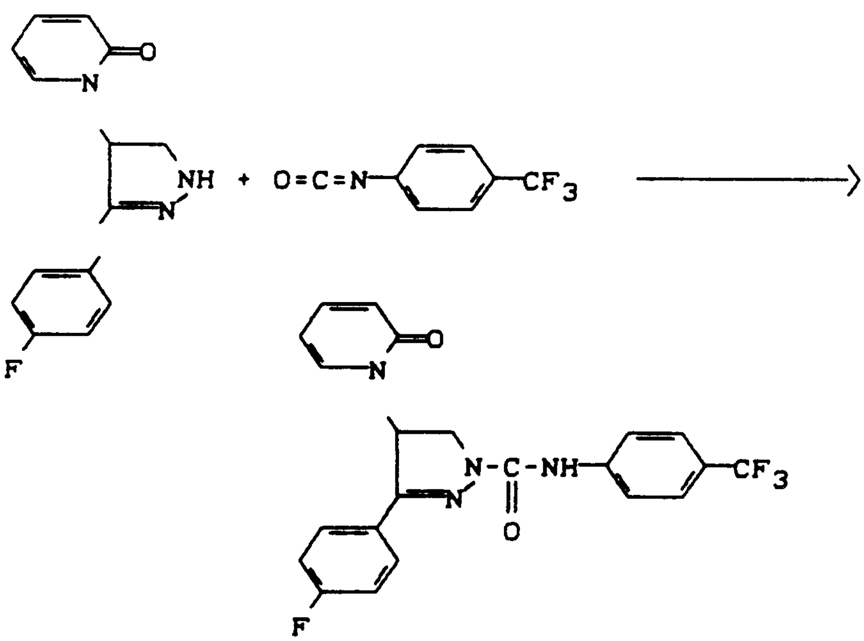

Verwendet man beispielsweise 3-(4-Chlorphenyl)-4,5-dihydro-1-pyrazolcarbonsäure-(4-trifluormethylanilid) und 2-Brompyridin als Ausgangsstoffe und n-Butyllithium, so läßt sich der Reaktionsablauf des erfindungsgemäßen Verfahrens (a-β) durch das folgende Formelschema darstellen:

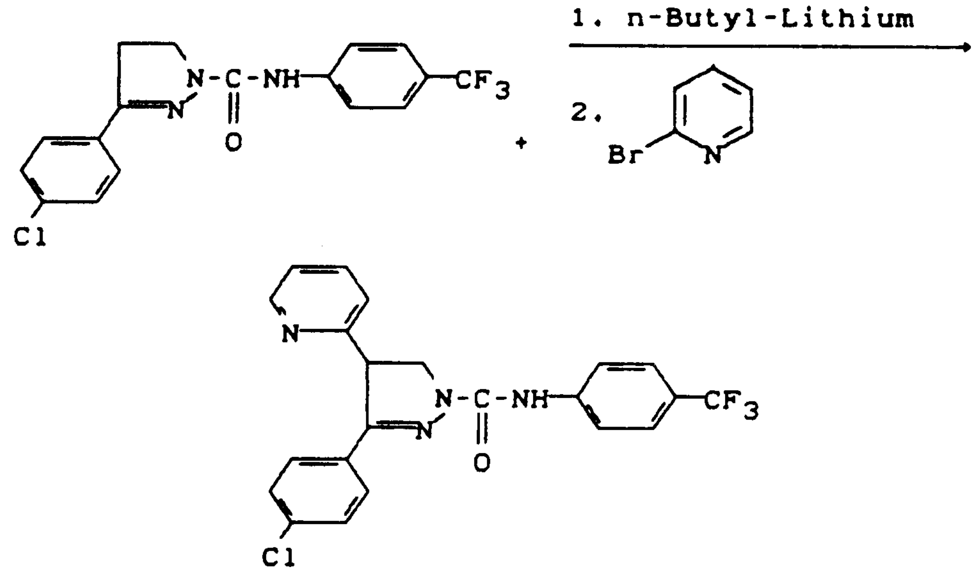


Verwendet man beispielsweise 3-(4-Fluorphenyl)-4-pyrid-2-on-1-yl-4,5-dihydro-1-pyrazolcarbonsäure-(4-trifluormethyl-anilid) und Methyliodid als Ausgangsverbindungen, so läßt sich der Reaktionsablauf des erfindungsgemäßen Verfahrens (b) durch das folgende Formelschema darstellen:

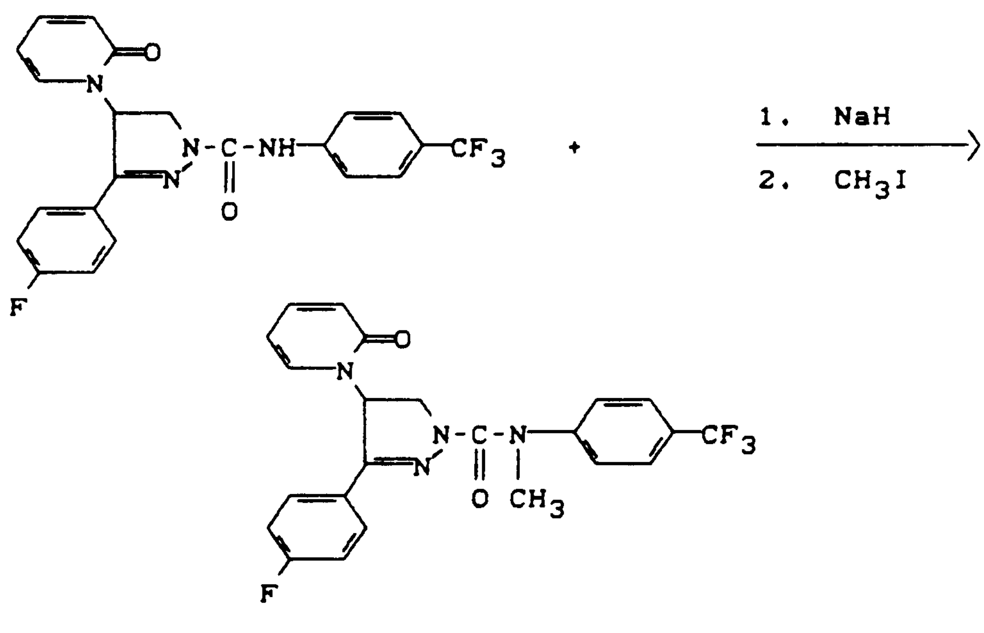


Die zur Durchführung des erfindungsgemäßen Verfahrens (a-α) als Ausgangsstoffe benötigten Pyrazolinderivate sind durch die Formel (II) allgemein definiert. In dieser Formel (II) stehen $R^1$, $R^2$, $R^3$, Y und Z vorzugsweise für diejenigen Reste, die bereits im Zusammenhang mit der Beschreibung der erfindungsgemäßen Stoffe der Formel (I) als bevorzugt für diese Substituenten genannt wurden. Die Pyrazolinderivate der Formel (II) sind neu.

Sie werden durch Umsetzung von Verbindungen der Formel (VII)

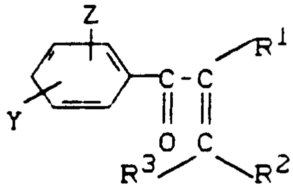

(VII)

in einem polaren organischen Lösungsmittel, vorzugsweise einem Alkanol bei Temperaturen von 20 bis 80 ° C insbesondere bei 30 bis 60 ° C mit Hydrazin-Hydrat hergestellt;

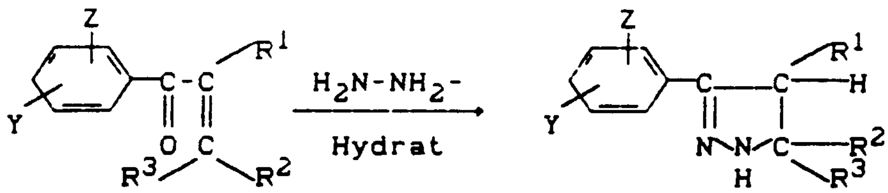


(VII) (II)

In Abhängigkeit von der Bedeutung der Substituenten $R^2$ und $R^3$ ergeben sich dabei folgende Herstellungsvarianten der Ausgangsverbindungen der Formel (VII)

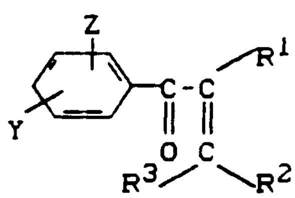

(VII)

a) $R^2$ und $R^3$ in der Formel (VII) stehen für Wasserstoff

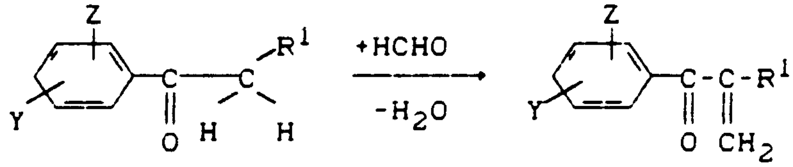


(VIII) (VIIa)

Hierbei wird in einem polaren organischen Lösungsmittel, vorzugsweise einem Alkanol und insbesondere in Ethanol oder Methanol mit einer Formalinlösung unter Zusatz geringer Mengen einer organischen Base, insbesondere von Piperidin sowie Zusatz von Eisessig umgesetzt.

b) In der Formel (VII) steht $R^2$ für Alkyl oder Aryl und $R^3$ steht für Wasserstoff:

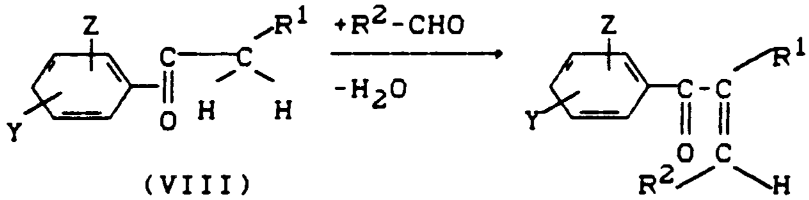


(VIII) (VIIb)

Die Verfahrensbedingungen entsprechen denjenigen der Umsetzung mit Formaldehyd.
c) In der Formel stehen $R^2$ und $R^3$ für Alkyl oder Aryl:

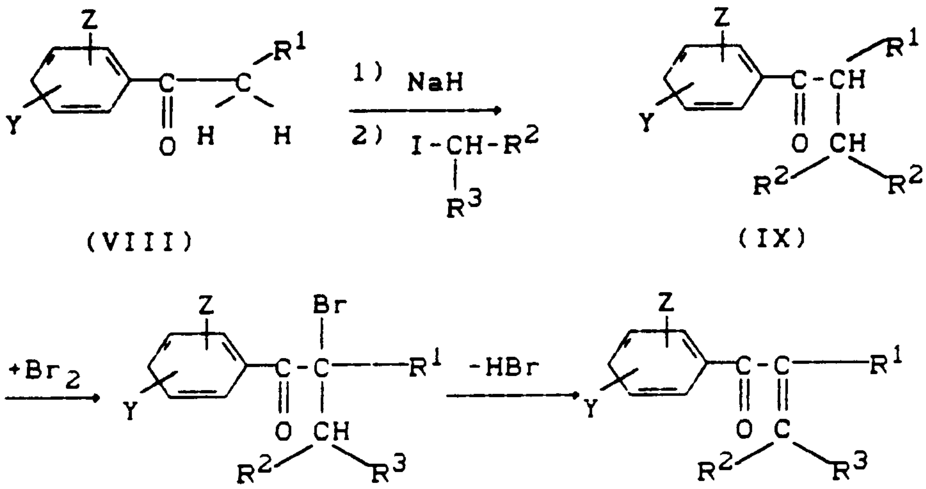


(VIII) (IX)

(X) (VIIc)

Hierbei wird die Verbindung (VIII) zunächst mit einer starken Base in das Salz übergeführt und anschließend mit einem Halogenid, insbesondere einem Iodid der Formel (XI)

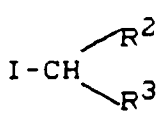

(XI)

umgesetzt.

Die dabei entstehende Verbindung der Formel (IX) wird bromiert und anschließend wird durch Zusatz einer Base unter HBr-Eliminierung das Zwischenprodukt der Formel (VIIc) hergestellt.

Alternativ können Verbindungen der Formel (II) in der $R^2$ und $R^3$ für Wasserstoff stehen auch hergestellt werden durch Umsetzung von Verbindungen der Formel (XII) in einem polaren Lösungsmittel, vorzugsweise in einem Alkanol bei Temperaturen von 0° bis 60°C mit Hydrazinhydrat:

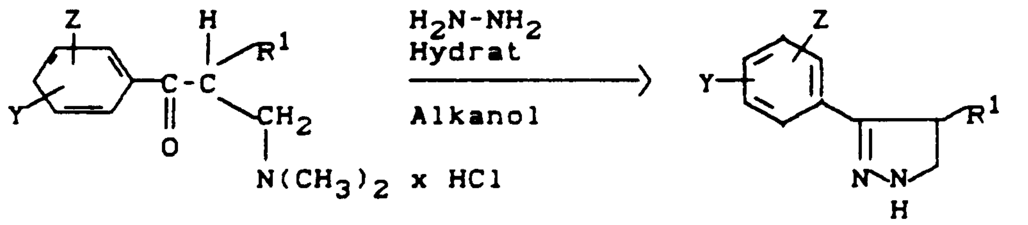


(XII) (II)

Verbindungen der Formel (XII) können hergestellt werden durch Umsetzung von Verbindungen der Formel (VIII) in einem Alkanol bei 30 bis 80°C mit Dimethylaminhydrochlorid und Paraformaldehyd und anschließendes Fällen des Salzes mit einem unpolaren Lösungsmittel z.B. Ether;

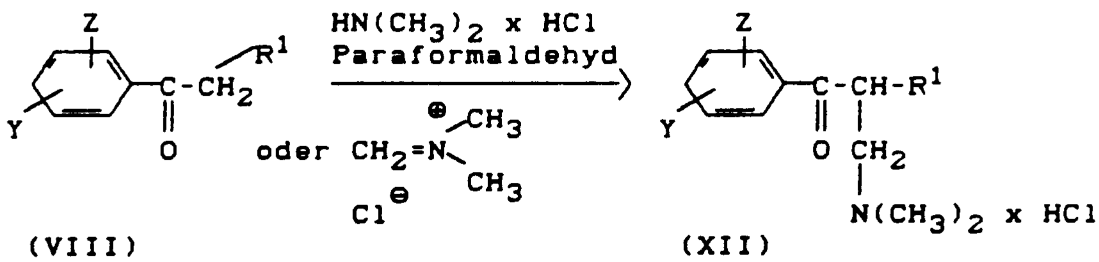


(VIII) (XII)

oder durch Umsetzung von Verbindungen der Formel (VIII) mit N,N-Dimethylmethylenimmoniumchlorid.

Die Verbindungen der Formel (VIII) sind allgemein bekannte Verbindungen der organischen Chemie und/oder können nach im Prinzip bekannten Methoden in einfacher Weise analog hergestellt werden (vgl. Herstellungsbeispiele).

Die Verbindungen der Formel (VII) sind teilweise bekannt und können nach den oben angegebenen Verbindungen hergestellt werden (vgl. Herstellungsbeispiele).

Die zur Durchführung des erfindungsgemäßen Verfahrens (a-α) weiterhin als Ausgangsstoffe benötigten Isocyanate bzw. Isothiocyanate der Formel (III) sind allgemein bekannte Verbindungen der organischen Chemie.

Beispielhaft, aber nicht einschränkend, seien außer den bei den Herstellungsbeispielen genannten Verbindungen die folgenden in Tabelle 2 aufgeführten Vorprodukte der allgemeinen Formel (II) genannt:

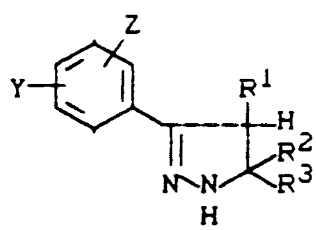

(II)

Tabelle 2:

| $R^1$ | $R^2$ | $R^3$ | Y | Z |
|---|---|---|---|---|
| 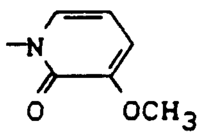 | H | H | H | 4-Cl |

Tabelle 2 - Fortsetzung:

| $R^1$ | $R^2$ | $R^3$ | Y | Z |
|---|---|---|---|---|
| 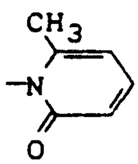 | H | H | H | 4-F |
| 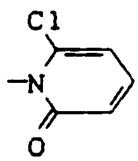 | H | H | H | 4-$OCHF_2$ |
| 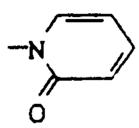 | H | H | H | H |
| 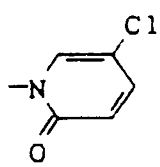 | H | H | H | 4-Br |
| 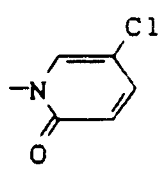 | H | H | H | 4-$OCHF_2$ |
| 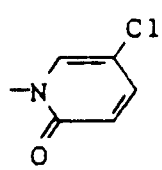 | H | H | H | 4-Cl |

Tabelle 2 - Fortsetzung:

| $R^1$ | $R^2$ | $R^3$ | Y | Z |
|---|---|---|---|---|
| 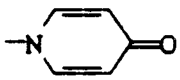 | H | H | H | 4-Cl |
| 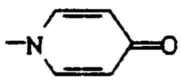 | H | H | H | 4-F |
| 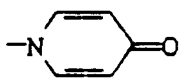 | H | H | H | 4-$OCHF_2$ |
| 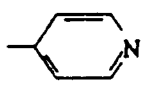 | H | H | H | 4-Cl |
| 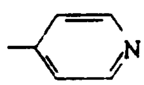 | H | H | H | 4-$OCHF_2$ |

Die zur Durchführung des erfindungsgemäßen Verfahrens (a-$\beta$) als Ausgangsstoffe benötigten Pyrazolcarbonsäureanilide sind durch die Formel (IV) allgemein definiert. In dieser Formel (IV) stehen $R^2$, $R^3$, $R^5$, X, Y und Z vorzugsweise für diejenigen Reste, die bereits im Zusammenhang mit der Beschreibung der erfindungsgemäßen Stoffe der Formel (I) als bevorzugt für diese Substituenten genannt wurden. Die Pyrazolcarbonsäureanilide der Formel (IV) sind bekannt (vgl. DE-OS 23 04 584).

Die zur Durchführung des erfindunggsgemäßen Verfahrens (a-$\beta$) weiterhin benötigten Halogenide der Formel (V) sowie die zur Durchführung des erfindungsgemäßen Verfahrens (b) benötigten Alkylierungsmittel der Formel (VI) sind allgemein bekannte Verbindungen der organischen Chemie.

Die zur Durchführung des erfindungsgemäßen Verfahrens (b) als Ausgangsstoffe benötigten Pyrazolinderivate sind neu und Gegenstand der Erfindung. Sie sind erhältlich nach Verfahren (a-$\alpha$) und (a-$\beta$).

Bei der Verfahrensvariante (a-$\alpha$)

werden Verbindungen der Formel (II)

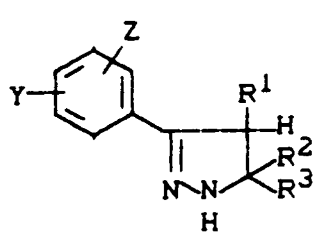

(II)

in welcher

$R^1$, $R^2$, $R^3$, Y und Z die oben angegebene Bedeutung haben,

gegebenenfalls in Gegenwart eines inerten organischen Lösungsmittels, wie beispielsweise Diethylether oder Acetonitril und gegebenenfalls in Gegenwart einer Base, wie beispielsweise Triethylamin, mit Isocyanaten bzw. Isothiocyanaten der Formel (III)

$X=C=N-R^5$ (III)

in welcher

X und $R^5$ die oben angegebene Bedeutung haben,

bei 0 bis 80 ° C umsetzt und in üblicher Weise aufgearbeitet (vgl. Herstellungsbeispiele).

Bei der Verfahrensvariante (a-$\beta$)

werden Verbindungen der Formel (IV)

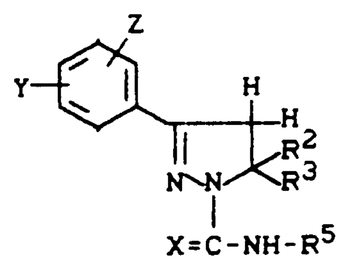

(IV)

in welcher

X, Y, Z, $R^2$, $R^3$ und $R^5$ die oben angegebene Bedeutung haben,

mit einer starken Base, vorzugsweise einer metallorganischen Verbindung, insbesondere mit Butyl-Lithium im inerten organischen Lösungsmittel bei Temperaturen von -50 bis 0 ° C, insbesondere von -30 ° C bis -15 ° C, gegebenenfalls in Anwesenheit einer Schutzgasatmosphäre, insbesondere Edelgasatmosphäre, wie z.B. Argon umsetzt und anschließend mit einem Halogenid

$Hal-R^1$

worin

$R^1$ und Hal die oben angegebene Bedeutung haben

bei -10 bis 60 ° C, insbesondere bei 0 bis -40 ° C umsetzt und durch Zusatz von Wasser und Extraktion mit Ether in üblicher Weise aufgearbeitet (vgl. Herstellungsbeispiele).

Bei der Verfahrensvariante (b)

werden Verbindungen der Formel (Ia)

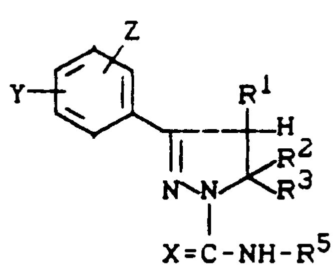

(Ia)

in welcher

X, Y, Z, $R^1$, $R^2$, $R^3$ und $R^5$ die oben angegebene Bedeutung haben,

gegebenenfalls in Gegenwart eines inerten organischen Lösungsmittels, wie beispielsweise Acetonitril, Dimethylformamid und Dimethylsulfoxid und in Gegenwart einer Base, wie beispielsweise Natriumcarbonat, Natriumhydroxid oder Triethylamin mit Halogenierungsmitteln der Formel (VI)

$R^4-E$ (VI)

in welcher

$R^4$ die oben angegebene Bedeutung hat und

E vorzugsweise für Halogen, insbesondere für Chlor, Brom oder Iod, oder für jeweils gegebenenfalls substituiertes Alkylsulfonyloxy oder Arylsulfonyloxy, wie beispielsweise Methansulfonyloxy, Trifluormethansulfonyloxy, Methoxysulfonyloxy, Ethoxysulfonyloxy oder p-Toluolsulfonyloxy steht,

bei Temperaturen zwischen 0 bis 100 °C umsetzt und in üblicher Weise aufarbeitet.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören;

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp.,

Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus. Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Tabakknospenraupe (Heliothis virescens); zur Bekämpfung von Bodeninsekten, wie beispielsweise gegen die Larven von Diabrotica balteata einsetzen.

Darüberhinaus lassen sie sich mit besonders gutem Erfolg zur Bekämpfung von parasitisch lebenden Warmblüterschädlingen, wie beispielsweise gegen die Larven der Goldfliege (Lucilia cuprina), gegen Musca domestica, gegen Periplaneta americana und Sitophilus granarius einsetzen.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage; z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder

Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die nachfolgenden Beispiele beschreiben die Herstellung und die Verwendung erfindungsgemäßer Wirkstoffe ohne sie darauf zu beschränken.

Herstellungsbeispiele

Beispiel 1

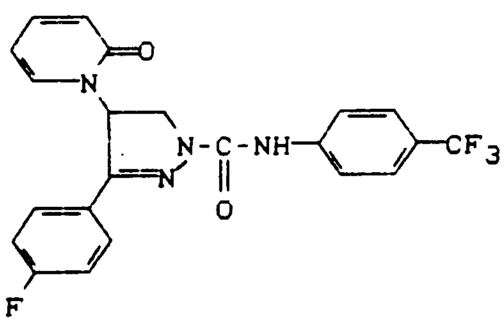

[Verfahren (a-α)]

2,98 g (0,0116 Mol) 3-(4-Fluorphenyl)-4-pyrid-2-on-1-yl-4,5-dihydropyrazol werden in 20 ml wasserfreiem Acetonitril bei 60 °C gelöst und unter Rühren 2,2 g (0,0116 Mol) 4-Trifluormethyl-phenylisocyanat und 2 Tropfen Triethylamin zugegeben. Man läßt die Reaktionsmischung 8 Stunden bei Raumtemperatur stehen. Anschließend wird das Acetonitril im Vakuum abgezogen und der Rückstand mit 20 ml Ether versetzt. Die sich abscheidenden Kristalle werden abgesaugt.

Man erhält 2,5 g (49 % der Theorie) 3-(4-Fluorphenyl)-4-pyrid-2-on-1-yl-4,5-dihydro-1-pyrazolcarbonsäure-(4-trifluormethyl-anilid) als farblosen Feststoff mit dem Schmelzpunkt 204 °C.

Beispiel 2

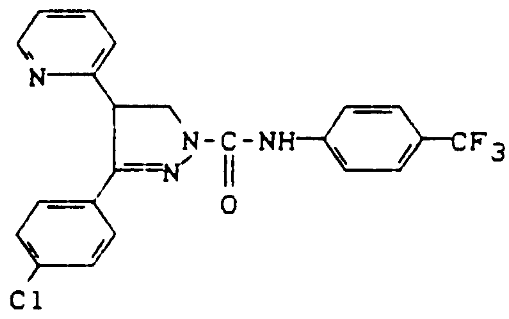

[Verfahren (a-β)]

8 g (0,0218 Mol) 3-(4-Chlorphenyl)-4,5-dihydro-1-pyrazolcarbonsäure-(4-trifluormethyl-anilid) werden in 80 ml wasserfreiem Tetrahydrofuran gelöst und unter Argonatmosphäre und Rühren bei -76 ° C 30 ml (0,048 Mol) n-Butyllithium-Lösung in Hexan (1,6 Mol pro Liter) zugetropft. Es wird 30 Minuten bei -76 ° C nachgerührt und dann bei -50 ° C 3,5 g (0,022 Mol) 2-Brompyridin gelöst in Tetrahydrofuran zugetropft. Man erwärmt auf Raumtemperatur, rührt noch 30 Minuten nach und versetzt dann die Reaktionsmischung mit 50 ml Wasser. Anschließend wird mit 100 ml Ether extrahiert, die Etherphase über Magnesiumsulfat getrocknet und dann das Lösungsmittel im Vakuum abgezogen. Der Rückstand wird mit 40 ml Ether verrührt und der entstandene kristalline Rückstand abgesaugt.

Man erhält 1,1 g (12 % der Theorie) 3-(4-Chlorphenyl)-4-pyrid-2-yl-4,5-dihydro-1-pyrazolcarbonsäure-(4-trifluormethyl-anilid) als hellgelben Feststoff mit dem Schmelzpunkt 219- 220 ° C.

In analoger Weise zu Beispiel 1 und 2 und unter Berücksichtigung der Angaben in der Beschreibung zu den erfindungsgemäßen Verfahren, werden die nachfolgend in Tabelle 3 aufgeführten Endprodukte der Formel (I) erhalten;

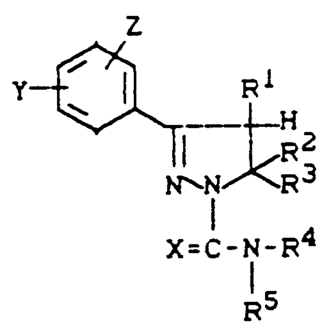

(I)

Tabelle 3:

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 3 | 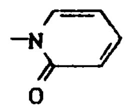 | H | H | H | 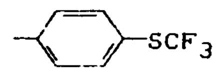 | O | H | 4-Cl | 253 |
| 4 | 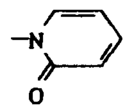 | H | H | H | 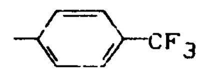 | O | H | 4-Cl | 206-208 |
| 5 | 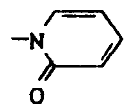 | H | H | H | 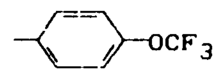 | O | H | 4-Cl | 226-228 |
| 6 | 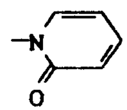 | H | H | H | 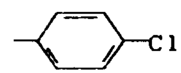 | O | H | 4-Cl | 217-220 |
| 7 | 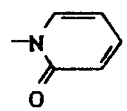 | H | H | H | 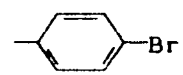 | O | H | 4-Cl | 209-211 |
| 8 | 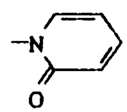 | H | H | H | 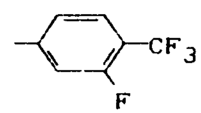 | O | H | 4-Cl | 197-199 |

<u>Tabelle 3</u> - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 9 | 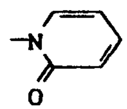 | H | H | H | 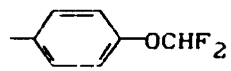 | O | H | 4-Cl | 196-198 |
| 10 | 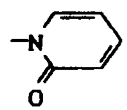 | H | H | H | 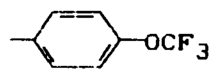 | O | H | 4-F | 208 |
| 11 | 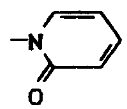 | H | H | H | 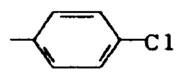 | O | H | 4-F | 215 |
| 12 | 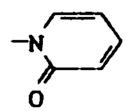 | H | H | H | 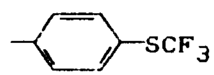 | O | H | 4-F | 226 |
| 13 | 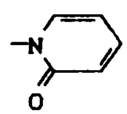 | H | H | H | 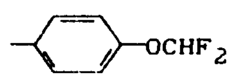 | O | H | 4-F | 182 |
| 14 | 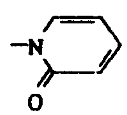 | H | H | H | 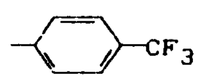 | O | H | 4-$OCHF_2$ | Öl |

**Tabelle 3** - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 15 | 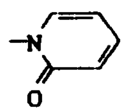 | H | H | H | 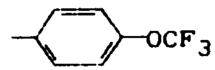 | O | H | 4-$OCHF_2$ | 172 |
| 16 | 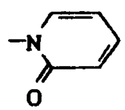 | H | H | H | 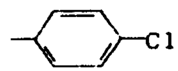 | O | H | 4-$OCHF_2$ | 212 |
| 17 | 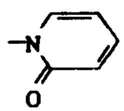 | H | H | H | 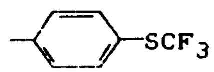 | O | H | 4-$OCHF_2$ | 185 |
| 18 | 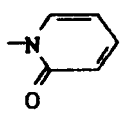 | H | H | H | 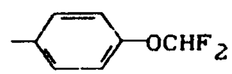 | O | H | 4-$OCHF_2$ | Öl |
| 19 | 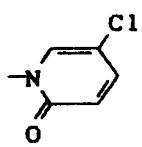 | H | H | H | 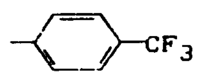 | O | H | 4-F | 228 |
| 20 | 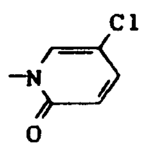 | H | H | H | 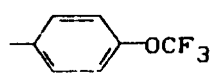 | O | H | 4-F | 228 |

**Tabelle 3** - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 21 | 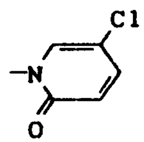 | H | H | H | 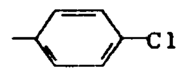 | O | H | 4-F | 222 |
| 22 | 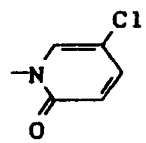 | H | H | H | 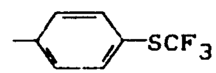 | O | H | 4-F | 236 |
| 23 | 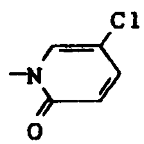 | H | H | H | 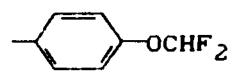 | O | H | 4-F | 204 |
| 24 | 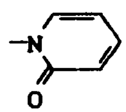 | H | H | H | 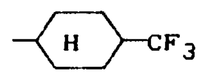 | O | H | 4-Cl | 186-188 |
| 25 | 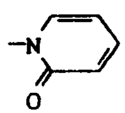 | H | H | H | 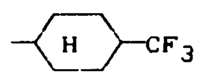 | O | H | 4-F | 168 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 26 | 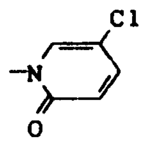 | H | H | H | 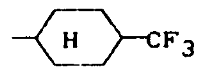 | O | H | 4-F | 207 |
| 27 | 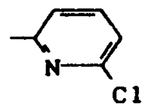 | H | H | H | 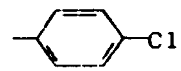 | O | H | $4-OC_2H_5$ | 182 |
| 28 | 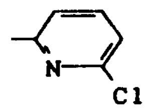 | H | H | H | 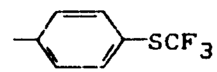 | O | H | $4-OC_2H_5$ | 161 |
| 29 | 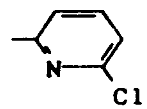 | H | H | H | 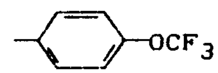 | O | H | $4-OC_2H_5$ | 182 |
| 30 | 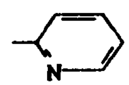 | H | H | H | 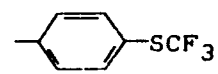 | O | H | $4-OCHF_2$ | 219 |
| 31 | 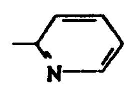 | H | H | H | 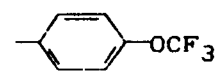 | O | H | $4-OCHF_2$ | 190 |

**Tabelle 3** - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 32 | 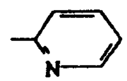 | H | H | H | 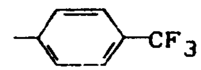 | O | H | 4-$OCHF_2$ | 196-197 |
| 33 | 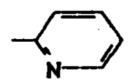 | H | H | H | 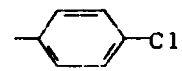 | O | H | 4-$OCHF_2$ | 217-220 |
| 34 | 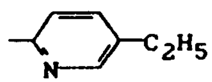 | H | H | H | 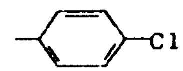 | O | H | 4-$OCHF_2$ | Öl |
| 35 | 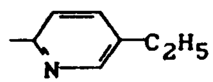 | H | H | H | 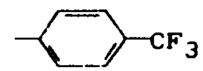 | O | H | 4-$OCHF_2$ | 148 |
| 36 | 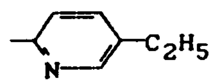 | H | H | H | 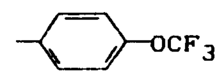 | O | H | 4-$OCHF_2$ | 169 |
| 37 | 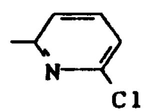 | H | H | H | 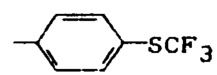 | O | H | 4-Cl | 201 |
| 38 | 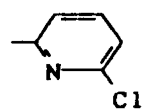 | H | H | H | 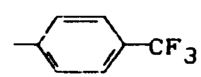 | O | H | 4-F | 214 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 39 | 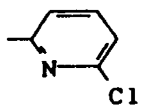 | H | H | H | 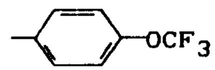 | O | H | 4-F | 213 |
| 40 | 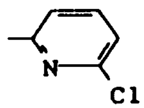 | H | H | H | 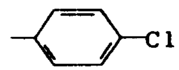 | O | H | 4-F | 198 |
| 41 | 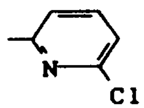 | H | H | H | 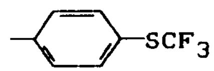 | O | H | 4-F | 201 |
| 42 | 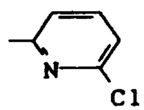 | H | H | H | 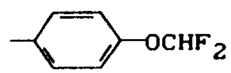 | O | H | 4-F | 221 |
| 43 | 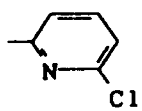 | H | H | H | 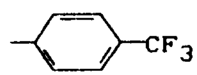 | O | H | 4-Br | 168 |
| 44 | 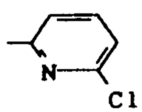 | H | H | H | 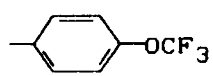 | O | H | 4-Br | 171 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 45 | 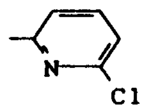 | H | H | H | 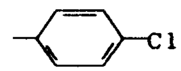 | O | H | 4-Br | 190 |
| 46 | 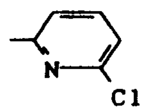 | H | H | H | 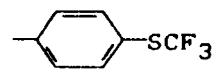 | O | H | 4-Br | 214 |
| 47 | 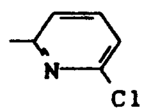 | H | H | H | 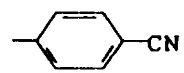 | O | H | 4-Br | 257 |
| 48 | 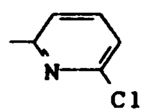 | H | H | H | 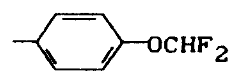 | O | H | 4-Br | 205 |
| 49 | 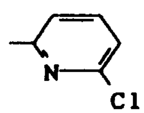 | H | H | H | 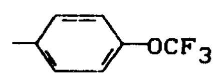 | O | H | 4-Cl | 171 |
| 50 | 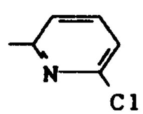 | H | H | H | 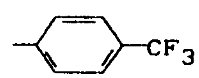 | O | H | 4-Cl | 198 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 51 | 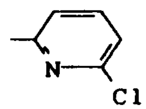 | H | H | H | 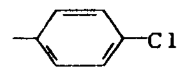 | O | H | 4-Cl | 226 |
| 52 | 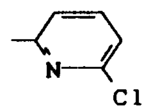 | H | H | H | 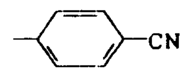 | O | H | 4-Cl | >250 |
| 53 | 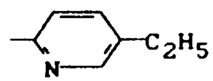 | H | H | H | 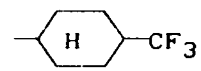 | O | H | $4-OCHF_2$ | 127-128 |
| 54 | 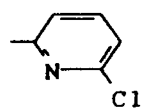 | H | H | H | 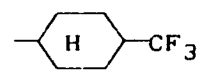 | O | H | 4-Br | 185 |
| 55 | 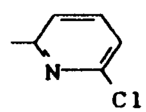 | H | H | H | 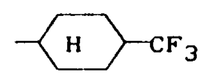 | O | H | 4-Cl | 221 |
| 56 | 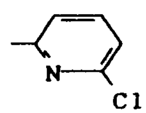 | H | H | H | 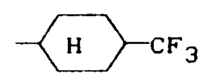 | O | H | 4-F | 167 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 57 | 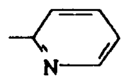 | H | H | H | 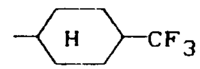 | O | H | 4-$OCHF_2$ | Harz |
| 58 | 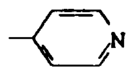 | H | H | H | 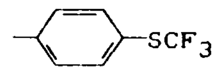 | O | H | 4-F | 184 |
| 59 | 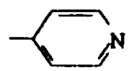 | H | H | H | 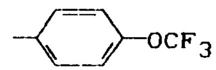 | O | H | 4-F | 155 |
| 60 | 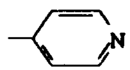 | H | H | H | 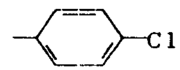 | O | H | 4-F | 166 |
| 61 | 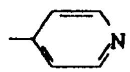 | H | H | H | 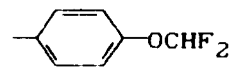 | O | H | 4-F | 148 |
| 62 | 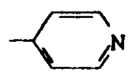 | H | H | H | 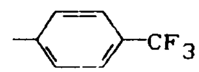 | O | H | 4-F | 191 |
| 63 | 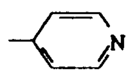 | H | H | H | 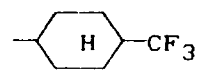 | O | H | 4-F | 192 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 64 | 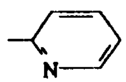 | H | H | H | 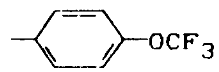 | O | H | 4-Cl | 215 |
| 65 | 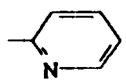 | H | H | H | 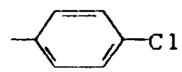 | O | H | 4-Cl | 260-262 |
| 66 | 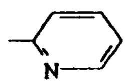 | H | H | H | 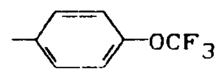 | O | H | 4-F | 205 |
| 67 | 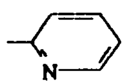 | H | H | H | 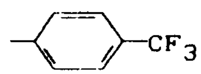 | O | H | 4-F | 198 |
| 68 | 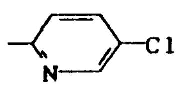 | H | H | H | 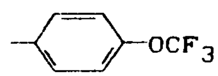 | O | H | 4-F | 178 |
| 69 | 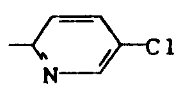 | H | H | H | 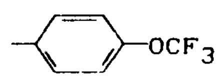 | O | H | 4-Cl | 198-200 |
| 70 | 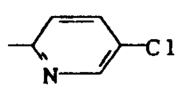 | H | H | H | 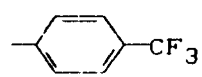 | O | H | 4-F | 180-182 |

<u>Tabelle 3</u> - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 71 | 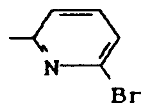 | H | H | H | 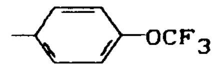 | O | H | 4-Cl | 193 |
| 72 | 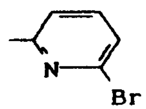 | H | H | H | 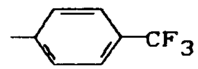 | O | H | 4-Cl | 219 |
| 73 | 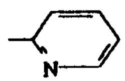 | H | H | H | 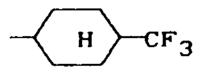 | O | H | 4-Cl | 204-206 |
| 74 | 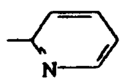 | H | H | H | 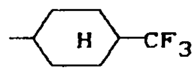 | O | H | 4-F | 207-209 |
| 75 | 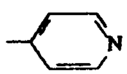 | H | H | H | 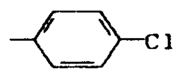 | O | H | 4-$OCHF_2$ | 184 |
| 76 | 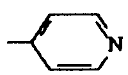 | H | H | H | 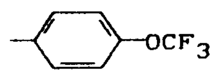 | O | H | 4-$OCHF_2$ | 162 |

Tabelle 3 - Fortsetzung

| Bsp. Nr. | $R^1$ | $R^2$ | $R^3$ | $R^4$ | $R^5$ | X' | Y | Z | physikal. Konstanten Fp. [°C] |
|---|---|---|---|---|---|---|---|---|---|
| 77 | 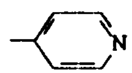 | H | H | H | 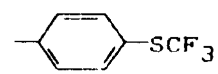 | O | H | 4-$OCHF_2$ | 159 |
| 78 | 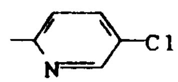 | H | H | H | 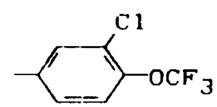 | O | H | 4-Cl | 205 |
| 79 | 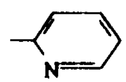 | H | H | H | 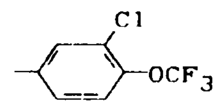 | O | H | 4-Cl | 204 |
| 80 | 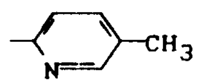 | H | H | H | 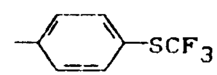 | O | H | 4-Cl | 230 |
| 81 | 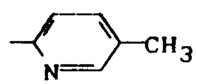 | H | H | H | 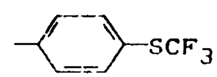 | O | H | 4-F | 228 |
| 82 | 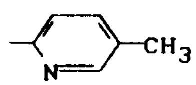 | H | H | H | 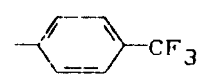 | O | H | 4-Cl | 196 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 83 | 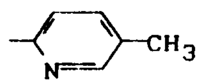 | H | H | H | 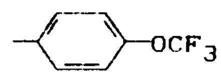 | O | H | 4-Cl | 222 |
| 84 | 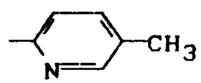 | H | H | H | 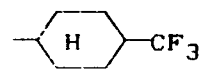 | O | H | 4-Cl | 174 |
| 85 | 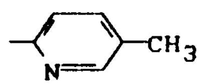 | H | H | H | 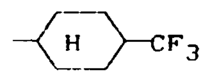 | S | H | 4-Cl | |
| 86 | 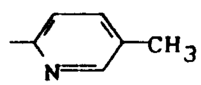 | H | H | H | 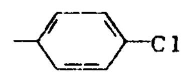 | O | H | 4-Cl | 182 |
| 87 | 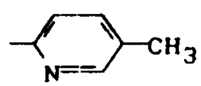 | H | H | H | 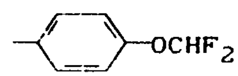 | O | H | 4-Cl | 168 |
| 88 | 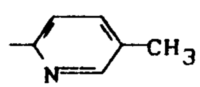 | H | H | H | 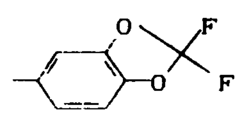 | O | H | 4-Cl | 218 |

<u>Tabelle 3</u> - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 89 | 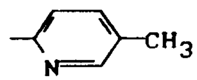 | H | H | H | 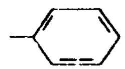 | O | H | 4-Cl | 187 |
| 90 | 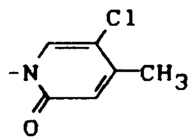 | H | H | H | 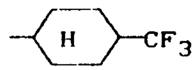 | O | H | 4-Cl | 198 |
| 91 | 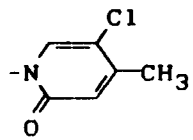 | H | H | H | 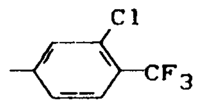 | O | H | 4-Cl | 203 |
| 92 | 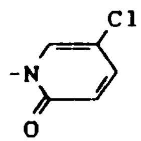 | H | H | H | 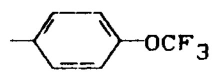 | O | H | 4-Cl | 243 |
| 93 | 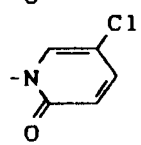 | H | H | H | 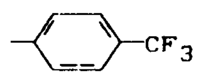 | O | H | 4-Cl | 193 |
| 94 | 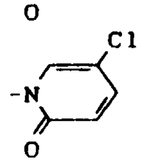 | H | H | H | 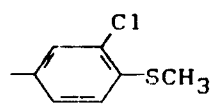 | O | H | 4-Cl | 203 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 95 | 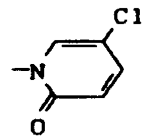 | H | H | H | 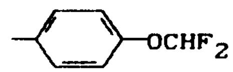 | O | H | 4-Cl | 223 |
| 96 | 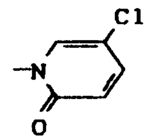 | H | H | H | 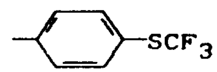 | O | H | 4-$OCHF_2$ | 210 |
| 97 | 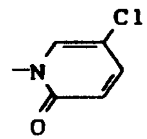 | H | H | H | 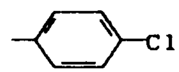 | O | H | 4-Cl | 178 |
| 98 | 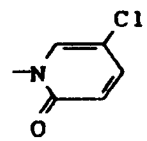 | H | H | H | 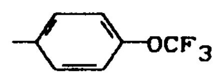 | O | H | 4-$OCHF_2$ | 201 |
| 99 | 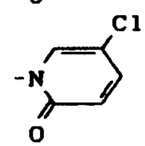 | H | H | H | 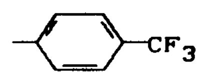 | O | H | 4-$OCHF_2$ | 197 |
| 100 | 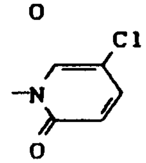 | H | H | H | 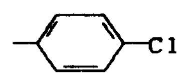 | O | H | 4-$OCHF_2$ | 178 |

<u>Tabelle 3</u> - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 101 | 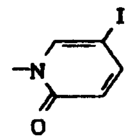 | H | H | H | 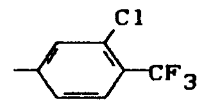 | O | H | 4-Cl | 235 |
| 102 | 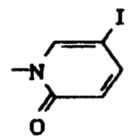 | H | H | H | 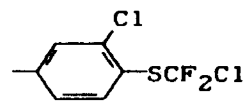 | O | H | 4-Cl | 244 |
| 103 | 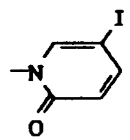 | H | H | H | 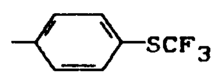 | O | H | 4-Cl | >250 |
| 104 | 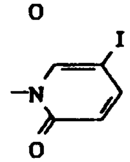 | H | H | H | 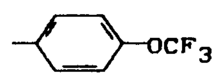 | O | H | 4-Cl | >250 |
| 105 | 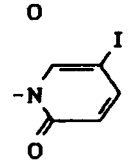 | H | H | H | 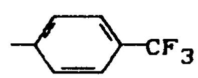 | O | H | 4-Cl | 234 |
| 106 | 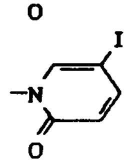 | H | H | H | 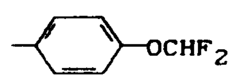 | O | H | 4-Cl | 243 |

<u>Tabelle 3</u> - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 107 | 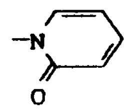 | H | H | H | 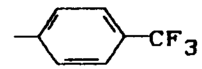 | O | 3,4-O-$CF_2$-O- | | 187 |
| 108 | 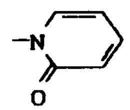 | H | H | H | 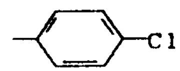 | O | 3,4-O-$CF_2$-O- | | 212 |
| 109 | 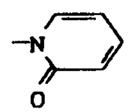 | H | H | H | 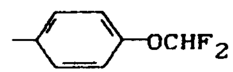 | O | 3,4-O-$CF_2$-O- | | 168 |
| 110 | 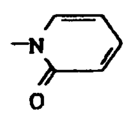 | H | H | H | 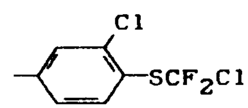 | O | 3,4-O-$CF_2$-O- | | 209 |
| 111 | 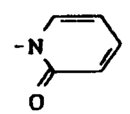 | H | H | H | 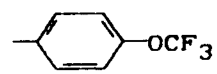 | O | 3,4-O-$CF_2$-O- | | 180 |
| 112 | 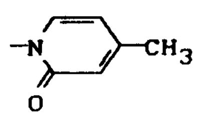 | H | H | H | 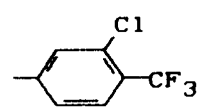 | O | H | 4-Cl | 212 |

**Tabelle 3** - Fortsetzung

| Bsp. Nr. | $R^1$ | $R^2$ | $R^3$ | $R^4$ | $R^5$ | X' | Y | Z | physikal. Konstanten Fp. [°C] |
|---|---|---|---|---|---|---|---|---|---|
| 113 | 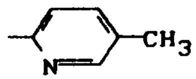 | H | H | H | 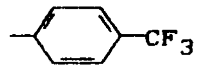 | O | H | 4-F | 188 |
| 114 | 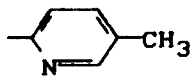 | H | H | H | 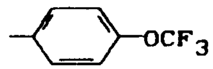 | O | H | 4-F | 180 |
| 115 | 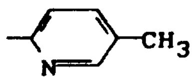 | H | H | H | 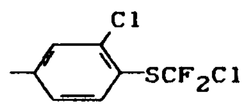 | O | H | 4-F | 185 |
| 116 | 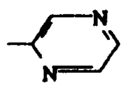 | H | H | H | 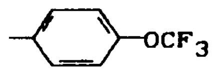 | O | H | 4-Cl | 224 |
| 117 | 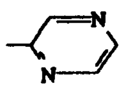 | H | H | H | 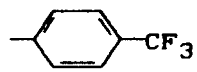 | O | H | 4-Cl | 208 |
| 118 | 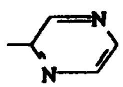 | H | H | H | 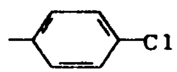 | O | H | 4-Cl | 209 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 119 | 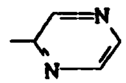 | H | H | H | 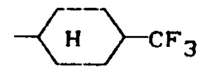 | O | H | 4-Cl | 185 |
| 120 | 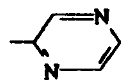 | H | H | H | 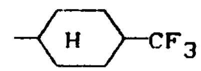 | S | H | 4-Cl | |
| 121 | 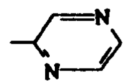 | H | H | H | 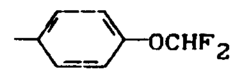 | O | H | 4-Cl | 205 |
| 122 | 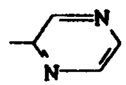 | H | H | H | 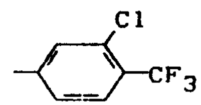 | O | H | 4-Cl | 233 |
| 123 | 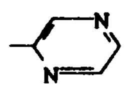 | H | H | H | 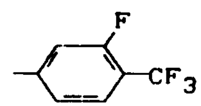 | O | H | 4-Cl | |
| 124 | 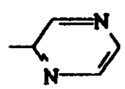 | H | H | H | 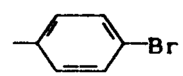 | O | H | 4-Cl | |
| 125 | 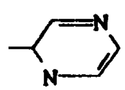 | H | H | H | 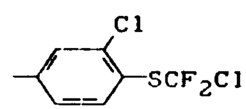 | O | H | 4-Cl | 218 |

**Tabelle 3** - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 126 | 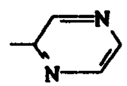 | H | H | H | 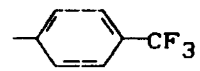 | O | H | 4-F | 185 |
| 127 | 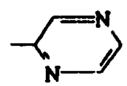 | H | H | H | 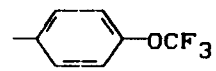 | O | H | 4-F | |
| 128 | 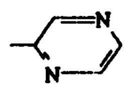 | H | H | H | 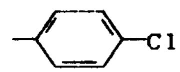 | O | H | 4-Cl | |
| 129 | 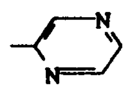 | H | H | H | 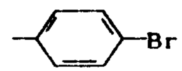 | O | H | 4-F | |
| 130 | 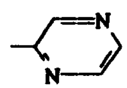 | H | H | H | 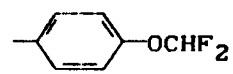 | O | H | 4-F | |
| 131 | 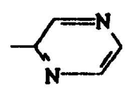 | H | H | H | 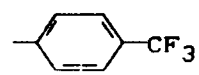 | O | H | 4-$OCHF_2$ | 198 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 132 | 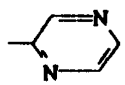 | H | H | H | 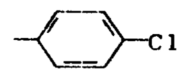 | O | H | 4-$OCHF_2$ | 212 |
| 133 | 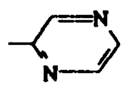 | H | H | H | 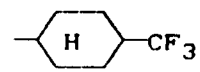 | O | H | 4-$OCHF_2$ | 158 |
| 134 | 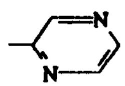 | H | H | H | 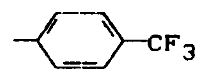 | O | H | 4-Br | 196 |
| 135 | 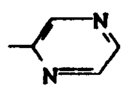 | H | H | H | 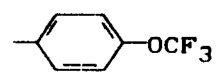 | O | H | 4-Br | 214 |
| 136 | 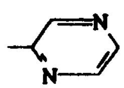 | H | H | H | 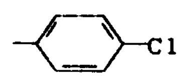 | O | H | 4-Cl | |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 137 | 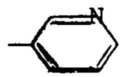 | H | H | H | 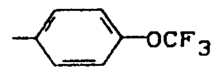 | O | H | H | 158 |
| 138 | 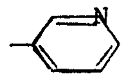 | H | H | H | 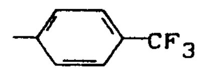 | O | H | H | 192 |
| 139 | 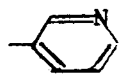 | H | H | H | 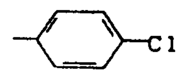 | O | H | H | 220 |
| 140 | 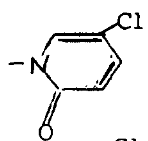 | H | H | H | 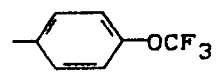 | O | H | H | 183 |
| 141 | 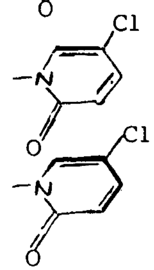 | H | H | H | 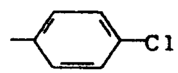 | O | H | H | 216 |
| 142 | | H | H | H | 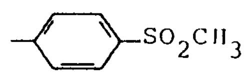 | O | H | H | 221 |
| 143 | 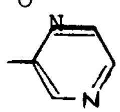 | H | H | H | 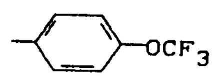 | O | 3-Cl | 4-Cl | 209 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 144 | 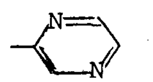 | H | H | H | 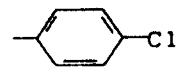 | O | 3-Cl | 4-Cl | 211 |
| 145 | 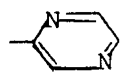 | H | H | H | 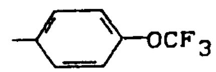 | O | H | 4-Br | 214 |
| 146 | 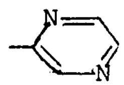 | H | H | H | 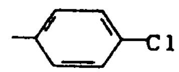 | O | H | 4-$CF_3$ | 229 |
| 147 | 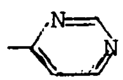 | H | H | H | 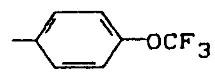 | O | H | 4-$OCHF_2$ | 207 |
| 148 | 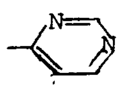 | H | H | H | 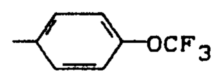 | O | H | 4-Br | 185 |
| 149 | 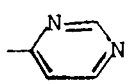 | H | H | H | 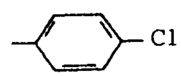 | O | H | 4-$OCH_3$ | 167 |
| 150 | 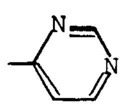 | H | H | H | 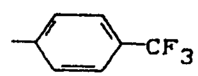 | O | H | 4-F | 171 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 151 | 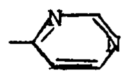 | H | H | H | 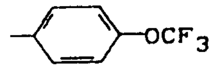 | O | H | H | 128 |
| 152 | 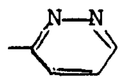 | H | H | H | 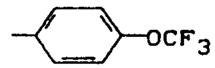 | O | H | 4-$OCHF_2$ | Öl |
| 153 | 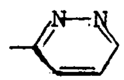 | H | H | H | 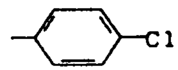 | O | H | 4-Cl | 219 |
| 154 | 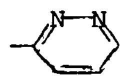 | H | H | H | 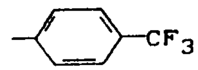 | O | H | 4-Cl | 213 |
| 155 | 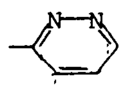 | H | H | H | 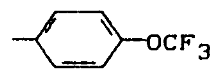 | O | H | 4-F | 193 |
| 156 | 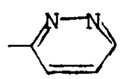 | H | H | H | 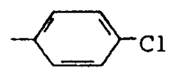 | O | H | 4-F | 202 |
| 157 | 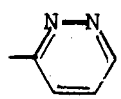 | H | H | H | 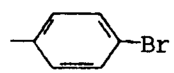 | O | H | 4-F | 243 |

<u>**Tabelle 3**</u> - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 158 | 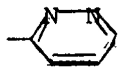 | H | H | H | 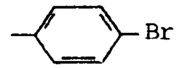 | O | H | 4-Cl | 204 |
| 159 | 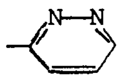 | H | H | H | 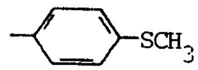 | O | H | 4-Cl | 151 |
| 160 | 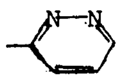 | H | H | H | 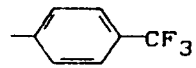 | 0 | H | H | 173 |
| 161 | 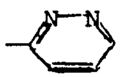 | H | H | H | 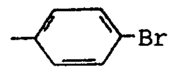 | O | H | H | 204 |
| 162 | 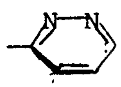 | H | H | H | 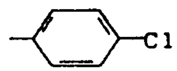 | O | H | H | 182 |
| 163 | 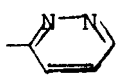 | H | H | H | 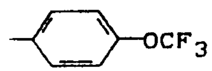 | O | H | H | 191 |
| 164 | 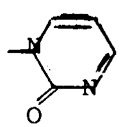 | H | H | H | 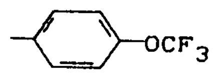 | O | H | 4-Cl | 227 |

**Tabelle 3** - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 165 | 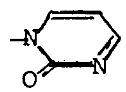 | H | H | H | 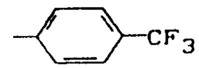 | O | H | 4-Cl | 234 |
| 166 | 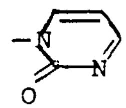 | H | H | H | 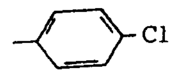 | O | H | 4-Cl | 224 |
| 167 | 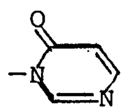 | H | H | H | 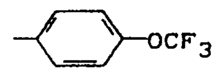 | O | H | 4-Cl | 198 |
| 168 | 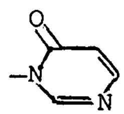 | H | H | H | 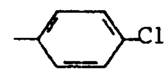 | O | H | 4-Cl | 212 |
| 169 | 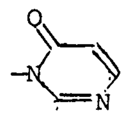 | H | H | H | 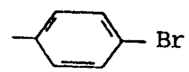 | O | H | 4-Cl | 234 |
| 170 | 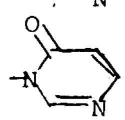 | H | H | H | 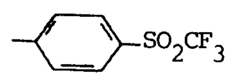 | O | H | 4-Cl | 161 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 171 | 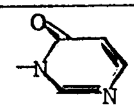 | H | H | H | 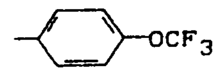 | O | H | H | 227 |
| 172 | 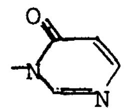 | H | H | H | 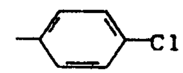 | O | H | H | 211 |
| 173 | 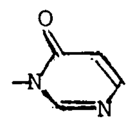 | H | H | H | 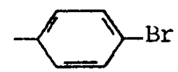 | O | H | H | 211 |
| 174 | 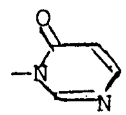 | H | H | H | 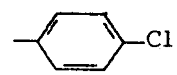 | O | H | 4-$OCHF_2$ | 197 |
| 175 | 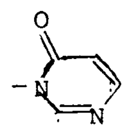 | H | H | H | 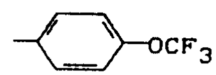 | O | H | 4-$OCHF_2$ | 143 |
| 176 | 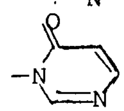 | H | H | H | 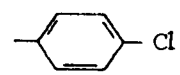 | O | H | 4-Br | 185 |
| 177 | 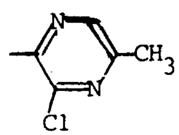 | H | H | H | 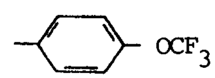 | O | H | 4-Cl | 244 |

Tabelle 3 - Fortsetzung

| Bsp. Nr. | $R^1$ | $R^2$ | $R^3$ | $R^4$ | $R^5$ | X | Y | Z | physikal. Konstanten Fp. [°C] |
|---|---|---|---|---|---|---|---|---|---|
| 178 | 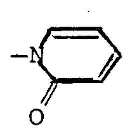 | H | H | H | 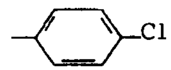 | O | H | 4-Br | |
| 179 | 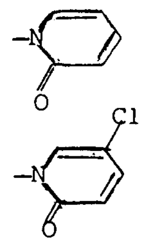 | H | H | H | 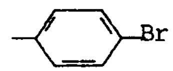 | O | H | 4-Br | |
| 180 | | H | H | H | 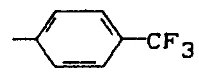 | O | H | 4-Br | |
| 181 | 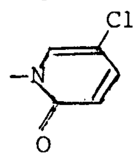 | H | H | H | 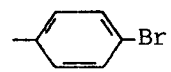 | O | H | 4-Br | |

## Herstellung der Vorprodukte

### Beispiel (II-1)

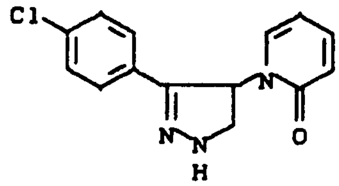

78,9 g (0,3 Mol) 4'-Chlor-2-pyrid-2-on-1-yl-acrylophenon werden in 300 ml Ethanol klar gelöst und unter Rühren 40 ml Hydrazinhydrat hinzugegeben. Man hält dabei die Temperatur bei 40-50 °C. Anschließend wird noch 1 Stunde bei 50 °C nachgerührt. Das Ethanol wird dann im Vakuum abdestilliert, der Rückstand mit 200 ml Wasser versetzt und mit 200 ml Ether extrahiert. Die abgetrennte Etherphase wird über Magnesiumsulfat getrocknet und dann der Ether im Vakuum abgezogen.

Man erhält 63,7 g (78 % der Theorie) 3-(4-Chlorphenyl)-4-pyrid-2-on-1-yl-4,5-dihydropyrazol als hellgelben Feststoff mit dem Schmelzpunkt 136 °C.

In analoger Weise zu Beispiel (II-1) und unter Berücksichtigung der Angaben in der Beschreibung zu den erfindungsgemäßen Verfahren, werden die nachfolgend in Tabelle 4 aufgeführten Vorprodukte der Formel (II) erhalten;

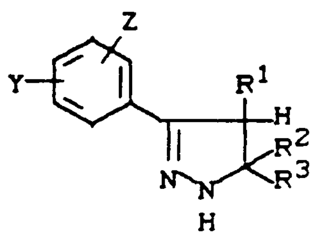

(II)

Tabelle 4:

| Bsp.-Nr. | $R^1$ | $R^2$ | $R^3$ | Y | Z | physikal. Konstante |
|---|---|---|---|---|---|---|
| II-2 | 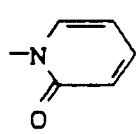 | H | H | H | 4-F | Öl |
| II-3 | 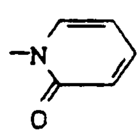 | H | H | H | 4-$OCHF_2$ | Öl |
| II-4 | 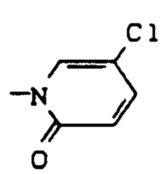 | H | H | H | 4-F | Öl |
| II-5 | 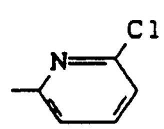 | H | H | H | 4-F | Öl |

Tabelle 4 - Fortsetzung:

| Bsp.-Nr. | $R^1$ | $R^2$ | $R^3$ | Y | Z | physikal. Konstante |
|---|---|---|---|---|---|---|
| II-6 | 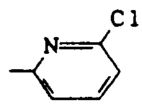 | H | H | H | 4-$OC_2H_5$ | Öl |
| II-7 | 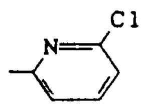 | H | H | H | 4-Br | Öl |
| II-8 | 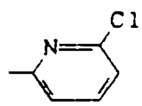 | H | H | H | 4-Cl | Öl |
| II-9 | 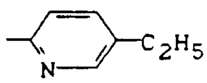 | H | H | H | 4-$OCHF_2$ | Öl |
| II-10 | 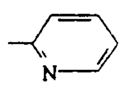 | H | H | H | 4-$OCHF_2$ | Öl |
| II-11 | 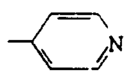 | H | H | H | 4-F | |
| II-12 | 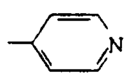 | H | H | H | 4-$OCHF_2$ | Öl |
| II-13 | 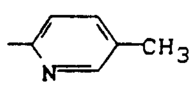 | H | H | H | 4-Cl | Öl |
| II-14 | 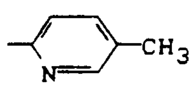 | H | H | H | 4-F | Öl |
| II-15 | 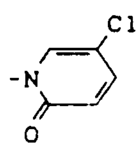 | H | H | H | 4-Cl | Öl |

Tabelle 4 - Fortsetzung:

| Bsp.-Nr. | $R^1$ | $R^2$ | $R^3$ | Y | Z | physikal. Konstante |
|---|---|---|---|---|---|---|
| II-16 | 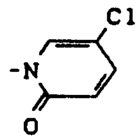 | H | H | H | 4-$OCHF_2$ | Öl |
| II-17 | 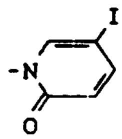 | H | H | H | 4-Cl | Öl |
| II-18 | 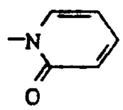 | H | H | 3,4-O-$CF_2$-O- | | Öl |
| II-19 | 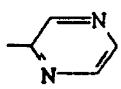 | H | H | H | 4-Cl | Öl |
| II-20 | 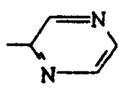 | H | H | H | 4-F | Öl |
| II-21 | 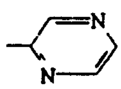 | H | H | H | 4-$OCHF_2$ | Öl |
| II-22 | 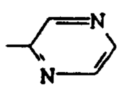 | H | H | H | 4-Br | Öl |
| II-23 | 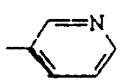 | H | H | H | H | Öl |
| II-24 | 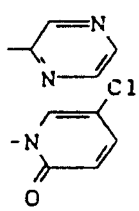 | H | H | H | 4-$CF_3$ | Schaum |
| II-25 | | H | H | H | H | Öl |

Tabelle 4 - Fortsetzung:

| Bsp.-Nr. | $R^1$ | $R^2$ | $R^3$ | Y | Z | physikal. Konstante |
|---|---|---|---|---|---|---|
| II-26 | 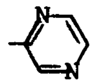 | H | H | 3-Cl | 4-Cl | Öl |
| II-27 | 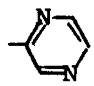 | H | H | H | $4-CF_3$ | Öl |
| II-28 | 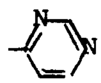 | H | H | H | $4-OCHF_2$ | Öl |
| II-29 | 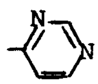 | H | H | H | 4-Br | Öl |
| II-30 | 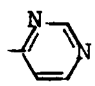 | H | H | H | $4-OCH_3$ | Öl |
| II-31 | 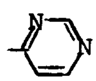 | H | H | H | 4-F | Öl |
| II-32 | 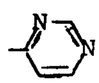 | H | H | H | H | Öl |
| II-33 | 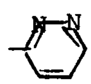 | H | H | H | $4-OCHF_2$ | Öl |
| II-34 | 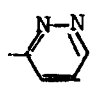 | H | H | H | 4-Cl | Öl |
| II-35 | 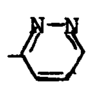 | H | H | H | 4-F | Öl |

Tabelle 4 - Fortsetzung:

| Bsp.-Nr. | $R^1$ | $R^2$ | $R^3$ | Y | Z | physikal. Konstante |
|---|---|---|---|---|---|---|
| II- 36 | 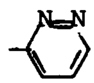 | H | H | H | H | Öl |
| II- 37 | 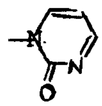 | H | H | H | 4-Cl | Öl |
| II- 38 | 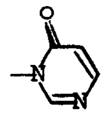 | H | H | H | 4-Cl | Öl |
| II- 39 | 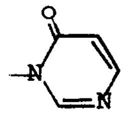 | H | H | H | H | Öl |
| II- 40 | 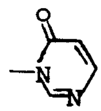 | H | H | H | $4\text{-}OCHF_2$ | Öl |
| II- 41 | 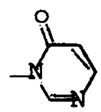 | H | H | H | 4-Br | Öl |
| II- 42 | 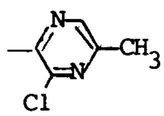 | H | H | H | 4-Cl | Öl |

Herstellung der Ausgangsprodukte

Beispiel (VIIa-1)

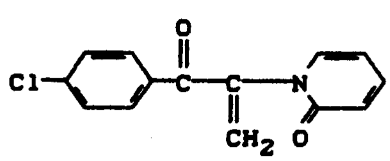

81,6 g (0,33 Mol) α-Pyrid-2-on-1-yl-4-chloracetophenon werden in 500 ml Methanol suspendiert. Anschließend werden bei 60 °C unter Rühren nacheinander 100 ml 37%ige Formalinlösung, 2 ml Piperidin und 2 ml Eisessig zugegeben. Es entsteht eine klare rotbraune Lösung, die noch 1 Stunde bei 60 °C nachgerührt wird. Die Reaktionsmischung wird dann im Vakuum eingeengt, der Rückstand mit 300 ml Wasser versetzt und mit 300 ml Ether extrahiert. Die abgetrennte Etherphase wird über Magnesiumsulfat getrocknet und anschließend der Ether im Vakuum abdestilliert.

Man erhält 78,9 g (92 % der Theorie) 4'-Chlor-2-pyrid-2-on-1-yl-acrylophenon als organgefarbenes Öl, das nach GC-MS eine Reinheit von 91 % hat. Das so erhaltene Öl wird ohne weitere Reinigung weiter umgesetzt.

In analoger Weise zu Beispiel (VIIa-1) und unter Berücksichtigung der Angaben in der Beschreibung zu den erfindungsgemäßen Verfahren, werden die nachfolgend in Tabelle 5 aufgeführten Ausgangsprodukte der Formel (VIIa) erhalten:

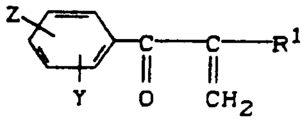

(VIIa)

Tabelle 5:

| Bsp.-Nr. | $R^1$ | Y | Z | physikal. Konstante |
|---|---|---|---|---|
| VIIa-2 | 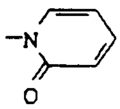 | H | 4-F | |
| VIIa-3 | 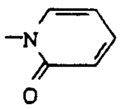 | H | 4-$OCHF_2$ | |
| VIIa-4 | 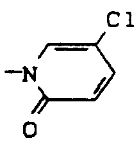 | H | 4-F | |
| VIIa-5 | 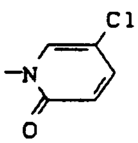 | H | 4-Cl | |

Tabelle 5 - Fortsetzung:

| Bsp.-Nr. | $R^1$ | Y | Z | physikal. Konstante |
|---|---|---|---|---|
| VIIa-6 | 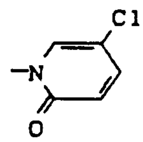 | H | H | |
| VIIa-7 | 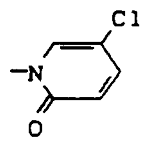 | H | 4-$OCHF_2$ | |
| VIIa-8 | 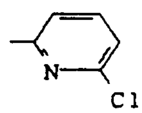 | H | 4-$OC_2H_5$ | |
| VIIa-9 | 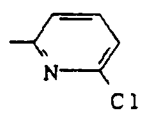 | 3,4-O-$CF_2$-O- | | |
| VIIa-10 | 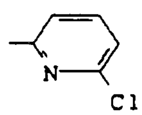 | H | 4-Cl | |
| VIIa-11 | 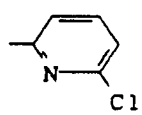 | H | 4-F | |
| VIIa-12 | 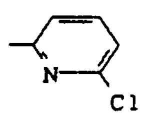 | H | 4-Br | |

Tabelle 5 - Fortsetzung:

| Bsp.-Nr.- | $R^1$ | Y | Z | physikal. Konstante |
|---|---|---|---|---|
| VIIa-13 | 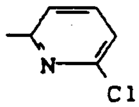 | H | 4-$OCHF_2$ | |
| VIIa-14 | 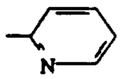 | 3,4-O-$CF_2$-O- | | |
| VIIa-15 | 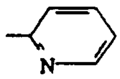 | H | 4-$OCHF_2$ | |
| VIIa-16 | 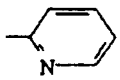 | H | 4-Br | |
| VIIa-17 | 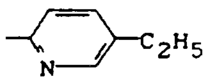 | H | 4-$OCHF_2$ | |
| VIIa-18 | 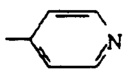 | H | 4-F | |
| VIIa-19 | 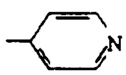 | H | 4-$OCHF_2$ | |
| VIIa-20 | 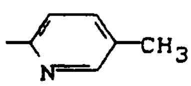 | H | 4-Cl | |
| VIIa-21 | 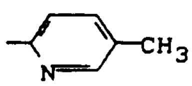 | H | 4-F | |

Tabelle 5 - Fortsetzung:

| Bsp.-Nr.- | $R^1$ | Y | Z | physikal. Konstante |
|---|---|---|---|---|
| VIIa-22 | 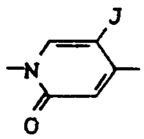 | H | 4-Cl | |
| VIIa-23 | 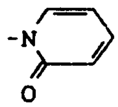 | 3,4-O-$CF_2$-O- | | |
| VIIa-24 | 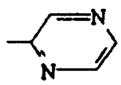 | H | 4-Cl | |
| VIIa-25 | 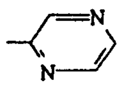 | H | 4-F | |
| VIIa-26 | 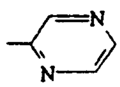 | H | 4-$OCHF_2$ | |
| VIIa-27 | 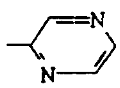 | H | 4-Br | |
| VIIa-28 | 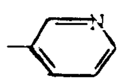 | H | H | |
| VIIa-29 | 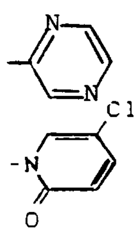 | H | 4-$CF_3$ | |
| VIIa-30 | | H | H | |
| VIIa-31 | 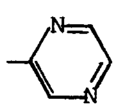 | 3-Cl | 4-Cl | |

Tabelle 5 - Fortsetzung:

| Bsp.-Nr. | $R^1$ | Y | Z | physikal. Konstante |
|---|---|---|---|---|
| VIIa- 32 | 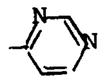 | H | 4-$OCHF_2$ | Öl |
| VIIa- 33 | 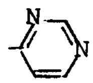 | H | 4-Br | Öl |
| VIIa- 34 | 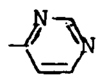 | H | 4-$OCH_3$ | Öl |
| VIIa- 35 | 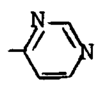 | H | 4-F | Öl |
| VIIa-36 | 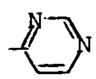 | H | H | Öl |
| VIIa- 37 | 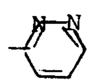 | H | 4-$OCHF_2$ | Öl |
| VIIa-38 | 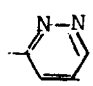 | H | 4-Cl | Öl |
| VIIa-39 | 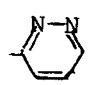 | H | 4-F | Öl |
| VIIa-40 | 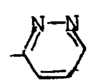 | H | H | Öl |

Tabelle 5 - Fortsetzung:

| Bsp.-Nr. | $R^1$ | Y | Z | physikal. Konstante |
|---|---|---|---|---|
| VIIa-41 | 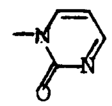 | H | 4-Cl | Öl |
| VIIa-42 | 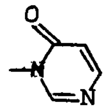 | H | 4-Cl | Öl |
| VIIa-43 | 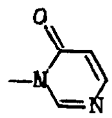 | H | H | Öl |
| VIIa-44 | 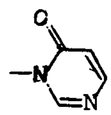 | H | $4-OCHF_2$ | Öl |
| VIIa-45 | 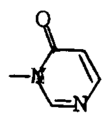 | H | 4-Br | Öl |
| VIIa-46 | 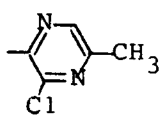 | H | 4-Cl | Öl |

Beispiel (VIII-1)

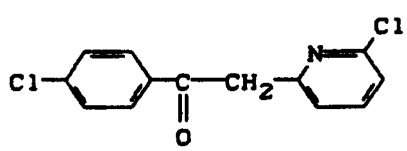

Unter Argonatmosphäre werden 25 g (0,2 Mol) 6-Chlor-2-picolin in 100 ml wasserfreiem Tetrahydrofuran gelöst. Bei -76 °C werden zu dieser Lösung 80 ml (0,2 Mol) n-Butyllithium-Lösung in n-Hexan (Gehalt 2,5 Mol/l) unter Rühren zugetropft. Anschließend wird die tiefrote Reaktionsmischung über einen Teflonschlauch mit Argonüberdruck in eine auf -76 °C gekühlte Lösung von 27,5 g (0,2 Mol) 4-Chlorbenzonitril in 100 ml wasserfreiem Tetrahydrofuran getropft. Man läßt auf Raumtemperatur aufwärmen, versetzt die Reaktionsmischung mit 200 ml Wasser, säuert mit wässriger Salzsäure an und rührt 1 Stunde. Anschließend wird die organische Phase abgetrennt, über Magnesiumsulfat getrocknet und das Lösungsmittel im Vakuum abgezogen.

Man erhält 43,6 g (82 % der Theorie) α-(6'-Chlorpyrid-2-yl)-4-chlor-acetephenon als gelben Feststoff.

Beispiel (VIII-2)

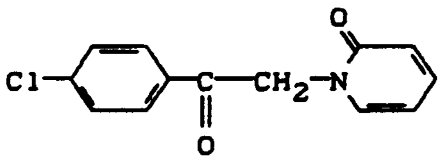

Unter Argonatmosphäre werden 1,6 g Natriumhydrid in 50 ml wasserfreiem Dimethylformamid suspendiert. Zu dieser Mischung werden 5 g (0,053 Mol) 2-Hydroxypyridin gelöst in 20 ml wasserfreiem Dimethylformamid langsam zugetropft. Anschließend wird 30 Minuten bei 60 °C nachgerührt. Bei 20 °C werden dann 12,4 g (0,053 Mol) 4-Chlorphenacylbromid gelöst in 50 ml wasserfreiem Dimethylformamid langsam zugetropft. Es wird 1 Stunde bei Raumtemperatur nachgerührt. Das Reaktionsgemisch wird im Vakuum eingeengt, der Rückstand mit 100 ml Wasser versetzt und mit 150 ml Ether extrahiert. Die abgetrennte Etherphase wird über Magnesiumsulfat getrocknet und der Ether im Vakuum abdestilliert.

Man erhält 13,2 g (100 % der Theorie) α-Pyrid-2-on-1-yl-4-chloracetophenon als hellbraunen Feststoff. Die Reinheit laut GC: 97 %.

Analog erhält man:

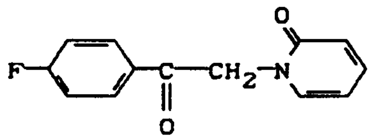
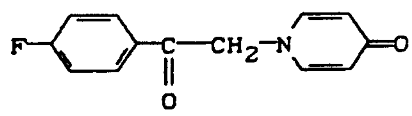
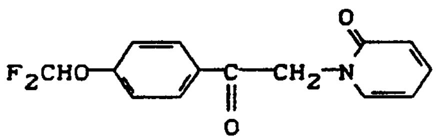
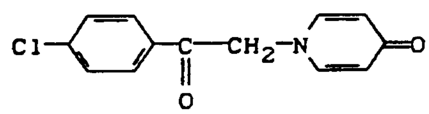
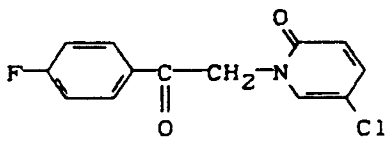
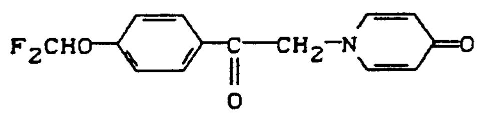
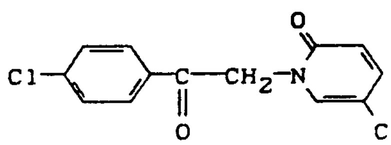
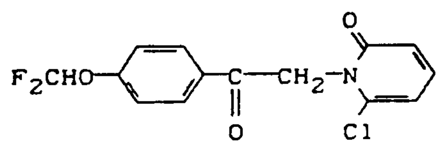
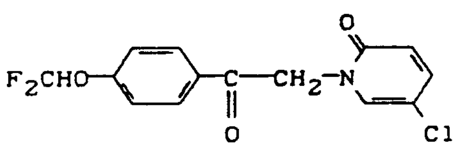
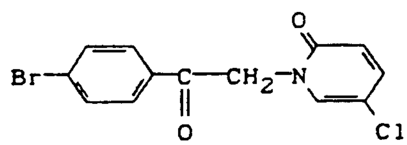
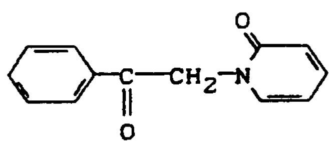
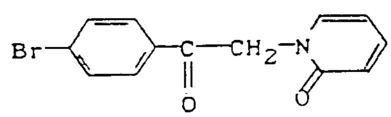
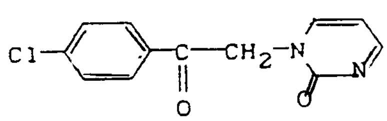
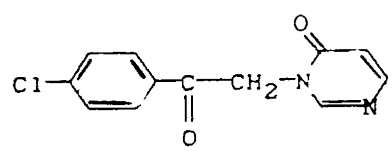

In analoger Weise zu den Beispielen (VIII-1) und (VIII-2) und unter Berücksichtigung der Angaben in der Beschreibung zu den erfindungsgemäßen Verfahren, werden die nachfolgend in Tabelle 6 aufgeführten Ausgangsprodukte der Formel (VIII) erhalten:

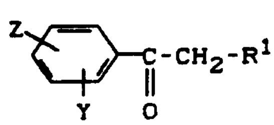
(VIII)

Tabelle 6:

| Bsp.-Nr. | $R^1$ | Y | Z |
|---|---|---|---|
| VIII-3 | 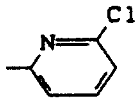 | H | 4-F |
| VIII-4 | 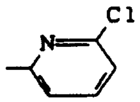 | H | $4-OC_2H_5$ |
| VIII-5 | 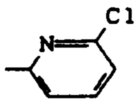 | H | 4-Br |
| VIII-6 | 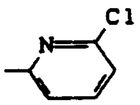 | H | $4-OCHF_2$ |

Tabelle 6 - Fortsetzung:

| Bsp.-Nr. | $R^1$ | Y | Z |
|---|---|---|---|
| VIII-7 | 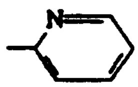 | H | 4-$OCHF_2$ |
| VIII-8 | 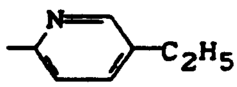 | H | 4-F |
| VIII-9 | 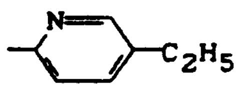 | H | 4-Cl |
| VIII-10 | 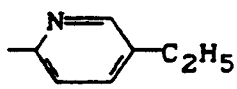 | H | 4-$OCHF_2$ |
| VIII-11 | 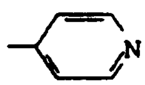 | H | 4-F |
| VIII-12 | 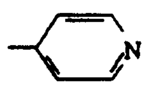 | H | 4-Cl |
| VIII-13 | 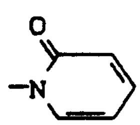 | H | 4-F |
| VIII-14 | 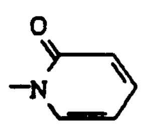 | H | 4-$OCHF_2$ |
| VIII-15 | 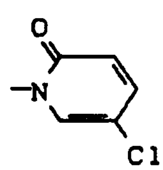 | H | 4-F |

<u>Tabelle 6</u> - Fortsetzung:

| Bsp.-Nr. | $R^1$ | Y | Z |
|---|---|---|---|
| VIII-16 | 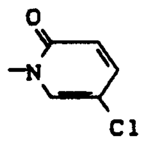 | H | 4-Cl |
| VIII-17 | 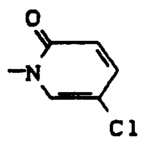 | H | $4\text{-}OCHF_2$ |
| VIII-18 | 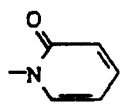 | H | H |
| VIII-19 | 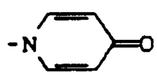 | H | 4-F |
| VIII-20 | 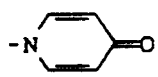 | H | 4-Cl |
| VIII-21 | 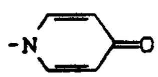 | H | $4\text{-}OCHF_2$ |
| VIII-22 | 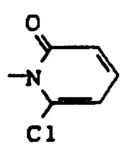 | H | $4\text{-}OCHF_2$ |
| VIII-23 | 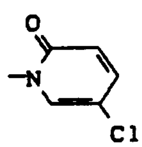 | H | 4-Br |

Tabelle 6 - Fortsetzung:

| Bsp.-Nr. | $R^1$ | Y | Z |
|---|---|---|---|
| VIII-24 | 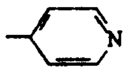 | H | 4-$OCHF_2$ |
| VIII-25 | 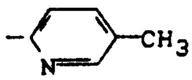 | H | 4-Cl |
| VIII-26 | 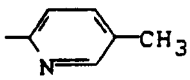 | H | 4-F |
| VIII-27 | 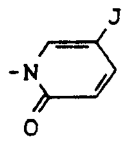 | H | 4-Cl |
| VIII-28 | 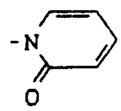 | 3,4-O-$CF_2$-O- | |
| VIII-29 | 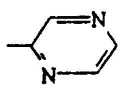 | H | 4-Cl |
| VIII-30 | 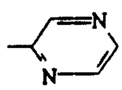 | H | 4-F |
| VIII-31 | 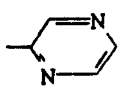 | H | 4-$OCHF_2$ |
| VIII-32 | 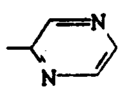 | H | 4-Br |

Tabelle 6 - Fortsetzung:

| Bsp.-Nr. | $R^1$ | Y | Z |
|---|---|---|---|
| VIII-33 | 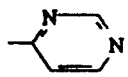 | H | 4-Cl |
| VIII-34 | 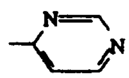 | H | 4-F |
| VIII-35 | 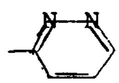 | H | 4-Cl |
| VIII-36 | 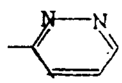 | H | 4-Br |
| VIII-37 | 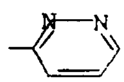 | H | $4-OCHF_2$ |
| VIII-38 | 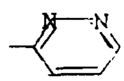 | H | H |
| VIII-39 | 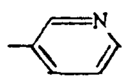 | H | H |

Anwendungsbeispiele

In den folgenden Anwendungsbeispielen wurden die nachstehend aufgeführten Verbindungen als Vergleichssubstanzen eingesetzt.

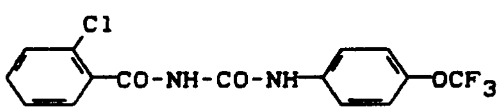 (A)

Triflumuron = 2-Chlor-N- [[[4-(trifluormethoxy)-phenyl]-amino]-carbonyl]-benzamid (bekannt aus: DE-A 2 601 780)

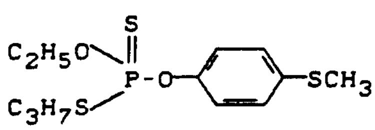 (B)

Sulprofos = O-(Ethyl-O-(4-methylthio)-phenyl)-S-propyldithiophosphat (bekannt aus: DE-A 2 111 414)

Beispiel A

Phaedon-Larven-Test

Lösungsmittel: 7 Gewichtsteile Dimethylformamid
Emulgator: 1 Gewichtateil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2, 4, 5, 6, 7, 8, 10, 11, 12, 31, 32, 38, 39, 40, 42, 43, 44, 54, 56, 62, 64, 66, 67, 68, 70 und 74.

Beispiel B

Heliothis virescens - Test

Lösungsmittel: 7 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik:
5, 8, 10, 25, 30, 31, 32, 37, 39, 41, 43, 44, 46, 54, 65, 68, 69, 70 und 74.

Beispiel C

Grenzkonzentrations-Test / Bodeninsekten

Testinsekt: Diabrotica balteata - Larven im Boden
Lösungsmittel: 4 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in 0,5 l Töpfe und läßt diese bei 20 ° C stehen.

Sofort nach dem Ansatz werden je Topf 5 vorgekeimte Maiskörner ausgelegt. Nach 1 Tag werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirksamkeit gegenüber dem Stand der Technik: 2, 66 und 67

Beispiel D

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1, 2, 5, 6, 7, 8, 9, 10, 11, 12, 14, 15, 16, 17, 24, 25, 38, 40, 41, 43, 44, 48, 54, 56, 59, 62, 65, 66, 67, 68, 69, 70, 73 und 74.

Beispiel: E

$LT_{100}$-Test

Testtiere: Periplaneta americana und Sitophilus granarius

Lösungsmittel:
35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (Ø 9,5 cm) pipettiert, die sich in Petri - schalen entsprechender Größe befinden. Nach Trocknung der Filterscheiben werden 5 Testtiere bei P. americana und ca. 30 Testtiere bei S. granarius in die Petri - schale überführt und diese wird abgedeckt.

Der Zustand der Testtiere wird bis zu 6 Stunden nach Ansetzen der Versuche kontrolliert. Es wird diejenige Zeit ermittelt, die für eine 100 %ige knock down-Wirkung erforderlich ist. Wird die $LT_{100}$ nach 6 Stunden nicht erreicht, wird der %-Satz der knock down gegangenen Testtiere festgestellt.

In diesem Test zeigten z.B. die Verbindungen der Herstellungsbeispiele 1, 2, 5, 7, 8, 9, 10, 13, 24, 25, 38, 39, 43, 44, 48, 54, 56, 62, 66, 68, 69, 70, 73 und 74 bei einer beispielhaften Konzentration von 1000 ppm a.i. eine $LT_{100}$ von 100 Minuten.

Beispiel: F

$LT_{100}$-Test

Testtiere: Musca domestica, Stamm WHO-N

Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (Ø 9,5 cm) pipettiert, die sich in Petri - schalen entsprechender Größe befinden. Nach Trocknung der Filterscheiben werden 25 Testtiere in die Petri - schale überführt und diese wird abgedeckt.

Der Zustand der Testtiere wird bis zu 6 Stunden laufend kontrolliert. Es wird diejenige Zeit ermittelt, die für eine 100 %ige knock down-Wirkung erforderlich ist. Wird die $LT_{100}$ nach 6 Stunden nicht erreicht, wird der %-Satz der knock down gegangenen Testtiere festgestellt.

In diesem Test zeigte z.B. die Verbindung des Herstellungsbeispiels 44 bei einer beispielhaften Konzentration von 1000 ppm a.i. eine $LT_{100}$ von 100 Minuten.

**Patentansprüche**

**1.** Substituierte 4-Hetaryl-pyrazoline der allgemeinen Formel (I)

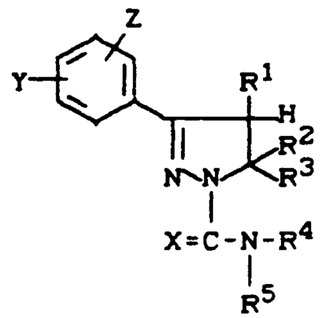

(I)

in welcher

$R^1$ für einen ungesättigten sechsgliedrigen, 1 bis 3 Stickstoffatome enthaltenden, gegebenenfalls substituierten und gegebenenfalls benzokondensierten Heterocyclus steht,

$R^2$ für Wasserstoff, Alkyl oder gegebenenfalls substituiertes Aryl steht,

$R^3$ für Wasserstoff oder Alkyl steht,

$R^4$ für Wasserstoff, Alkyl, Phenyl oder Alkylthio steht,

$R^5$ für gegebenenfalls substituiertes Alkyl, gegebenenfalls substituiertes Cycloalkyl oder für den Rest

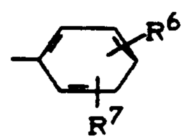

steht,

wobei $R^6$ und $R^7$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, Halogendialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl oder wobei $R^6$ und $R^7$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy, Halogenalkylthio, Alkylcarbonyl, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Amino, Nitro, Cyano oder wobei Y und Z zusammen für gegebenenfalls durch Halogen substituiertes 3,4-Methylendioxy oder 3,4-Ethylendioxy stehen.

**2.** Substituierte 4-Hetaryl-pyrazoline der allgemeinen Formel (I) gemäß Anspruch 1, in welcher

$R^1$ für einen gegebenenfalls durch Halogen, Hydroxy, Alkyl($C_1$-$C_6$), CN, $NO_2$, Alkoxy($C_1$-$C_6$)-carbonyl, Alkyl($C_1$-$C_4$)thio, Alkoxy($C_1$-$C_6$), Halogenalkyl($C_1$-$C_4$), Halogenalkyl($C_1$-$C_4$)thio, Halogenalkoxy($C_1$-$C_4$), Dialkyl-($C_1$-$C_4$)amino oder Dihalogenamino substituierten Pyridin-, Pyridon-, Pyrimidin-, Pyrimidon-, Pyridazinon-, Pyrazinon-, Pyrazin-, Pyridazin- oder Triazin-Rest steht,

$R^2$ für Wasserstoff, Alkyl($C_1$-$C_6$) oder gegebenenfalls einfach bis dreifach, gleich oder verschieden substituiertes Phenyl steht, wobei als Substituenten genannt seien: Halogen, Cyano, Alkyl($C_1$-$C_6$), Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio,

$R^3$ für Wasserstoff oder Alkyl($C_1$-$C_6$) steht,

$R^4$ für Wasserstoff, Alkyl($C_1$-$C_6$), Phenyl oder Alkyl($C_1$-$C_4$)thio steht,

$R^5$ für gegebenenfalls durch Halogen, Halogenalkyl($C_1$-$C_4$), Halogenalkoxy($C_1$-$C_4$) substituiertes

Alkyl($C_1$-$C_6$), für gegebenenfalls durch Halogen, Halogenalkyl($C_1$-$C_4$),Halogenalkoxy($C_1$-$C_4$) substituiertes Cycloalkyl ($C_3$-$C_7$) oder für den Rest

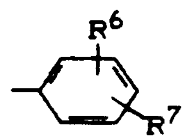

steht, wobei $R^6$ und $R^7$ gleich oder verschieden sein können und für Wasserstoff, Halogen, Alkyl-($C_1$-$C_6$), Nitro, Cyano, Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio, gegebenenfalls durch Halogen, Halogenalkyl($C_1$-$C_6$), Alkoxy-($C_1$-$C_6$), Alkyl($C_1$-$C_6$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_6$)carbonyl, Alkenyl($C_2$-$C_6$)oxy, Alkinyl($C_2$-$C_6$), Alkyl($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)-carbonyl, Alkyl ($C_1$-$C_4$)-carbonyl, stehen oder wobei

$R^6$ und $R^7$ zusammen für einen der folgenden bivalenten Reste stehen:

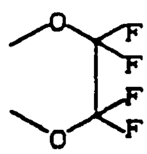 , 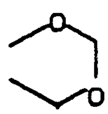 , 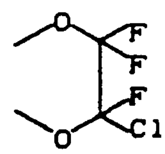 , 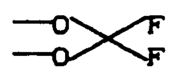 ,

X für Sauerstoff oder Schwefel steht und

Y und Z gleich oder verschieden sein können und für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_4$), Halogenalkyl($C_1$-$C_4$)thio, Alkyl($C_1$-$C_4$)carbonyl, Alkoxy($C_1$-$C_4$)carbonyl, Halogenalkoxy($C_1$-$C_4$)-carbonyl, gegebenenfalls durch Halogen, Alkyl($C_1$-$C_4$), Alkoxy($C_1$-$C_4$), Halogenalkyl ($C_1$-$C_4$) substituiertes Phenoxy oder Phenylthio, Alkenyl($C_2$-$C_6$)oxy, Alkinyl($C_2$-$C_6$), Alkyl($C_1$-$C_4$)-thionyl, Alkyl($C_1$-$C_4$)sulfonyl, Halogenalkyl($C_1$-$C_4$)thionyl, Halogenalkyl($C_1$-$C_4$)sulfonyl, gegebenenfalls durch Halogen, Alkoxy($C_1$-$C_4$), Halogenalkyl($C_1$-$C_4$) substituiertes Mono- oder Dialkyl($C_1$-$C_6$)amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

**3.** Substituierte 4-Hetaryl-pyrazoline der allgemeinen Formel (I) gemäß Anspruch 1, in welcher

$R^1$ für einen gegebenenfalls durch Fluor, Chlor, Brom, Iod, Hydroxy, Alkyl($C_1$-$C_4$), CN, $NO_2$, Alkoxy($C_1$-$C_4$)carbonyl, Alkyl($C_1$-$C_3$)thio, Alkoxy($C_1$-$C_4$), Halogenalkyl($C_1$-$C_3$), Halogenalkyl-($C_1$-$C_3$)thio oder Halogenalkoxy($C_1$-$C_4$)-carbonyl substituierten Pyridin-, Pyridon-, Pyrimidin-, Pyrimidon-, Pyridazinon-, Pyrazinon-, Pyrazin-, Pyridazin- oder Triazin-Rest steht,

$R^2$ für Wasserstoff, Alkyl($C_1$-$C_4$) oder gegebenenfalls durch Halogen, Cyano, Alkyl($C_1$-$C_3$), Halogenalkyl($C_1$-$C_3$), Alkoxy($C_1$-$C_3$), Halogenalkoxy($C_1$-$C_3$), Alkyl($C_1$-$C_3$)thio oder Halogenalkyl($C_1$-$C_3$)thio substituiertes Phenyl steht,

$R^3$ für Wasserstoff oder Alkyl($C_1$-$C_4$) steht,

$R^4$ für Wasserstoff, Alkyl($C_1$-$C_4$), Phenyl oder Alkyl($C_1$-$C_3$)thio steht,

$R^5$ für gegebenenfalls durch Fluor, Chlor, Brom, Halogenalkyl($C_1$-$C_3$), Halogenalkoxy($C_1$-$C_3$)-substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cycloalkyl($C_3$-$C_6$) oder für den Rest

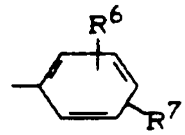

steht,

wobei $R^6$ und $R^7$ gleich oder verschieden sein können und für Wasserstoff, Fluor, Chlor, Brom, Iod, Alkyl($C_1$-$C_4$), Nitro, Cyano, Halogenalkyl($C_1$-$C_3$), Alkoxy-($C_1$-$C_4$), Halogenalkoxy ($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$), Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituierts Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$),Alkoxy($C_1$-$C_3$)carbonyl, Alkyl($C_1$-$C_3$)thionyl, Alkyl ($C_1$-$C_3$)carbonyl, Halogenalkyl ($C_1$-$C_3$)sulfuryl stehen oder wobei

$R^6$ und $R^7$ zusammen für einen der der folgenden bivalenten Reste stehen:

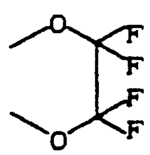 , 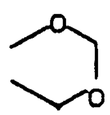 , 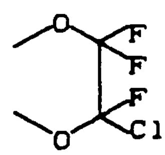 , 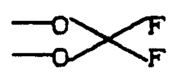 ,

X für Sauerstoff oder Schwefel steht und

Y und Z gleich oder verschieden sein können und für Wasserstoff, Alkyl($C_1$-$C_4$), Fluor, Chlor, Brom, Jod, Halogenalkyl($C_1$-$C_4$), Alkoxy($C_1$-$C_4$), Alkyl($C_1$-$C_4$)thio , Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Alkyl($C_1$-$C_3$), Alkoxy($C_1$-$C_3$), Halogenalkyl($C_1$-$C_3$) substituiertes Phenoxy oder Phenylthio, Halogenalkoxy($C_1$-$C_3$)carbonyl, Alkenyl($C_2$-$C_4$)oxy, Alkinyl($C_2$-$C_4$), Alkyl($C_1$-$C_3$) thionyl, Alkyl-($C_1$-$C_3$)sulfonyl, Halogenalkyl($C_1$-$C_3$) thionyl, Halogenalkyl($C_1$-$C_3$)sulfonyl, gegebenenfalls durch Halogen, Alkoxy ($C_1$-$C_3$), Halogenalkyl ($C_1$-$C_3$), substituiertes Mono- oder Dialkyl ($C_1$-$C_3$)-amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

**4.** Substituierte 4-Hetaryl-pyrazoline der allgemeinen Formel (I) gemäß Anspruch 1, in welcher

$R^1$ für einen gegebenenfalls durch Fluor, Chlor, Brom, Iod, Hydroxy, Alkyl($C_1$-$C_4$), CN, $NO_2$, Alkoxy($C_1$-$C_4$)carbonyl, Alkyl($C_1$-$C_3$)thio, Alkoxy($C_1$-$C_4$), Halogenalkyl($C_1$-$C_3$), Halogenalkyl($C_1$-$C_3$)thio,Halogenalkoxy($C_1$-$C_4$) oder Halogenalkoxy($C_1$-$C_4$)-carbonyl substituierten

Pyridin-, Pyridon-, Pyrimidin-, Pyrimidon-, Pyridazinon-, Pyrazinon- Pyrazin- oder Pyridazinrest steht,

$R^2$ für Wasserstoff oder Alkyl($C_1$-$C_4$) steht,

$R^3$ für Wasserstoff oder Alkyl($C_1$-$C_4$) steht,

$R^4$ für Wasserstoff oder Alkyl($C_1$-$C_4$) steht und

$R^5$, X und Z die in Anspruch 3 angegebene Bedeutung besitzen.

**5.** Verfahren zur Herstellung von substituierten 4-Hetaryl-pyrazolinen der allgemeinen Formel (I)

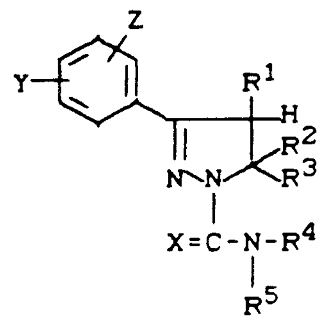

(I)

in welcher

- $R^1$ für einen ungesättigten sechsgliedrigen, 1 bis 3 Stickstoffatome enthaltenden, gegebenenfalls substituierten und gegebenenfalls benzokondensierten Heterocyclus steht,
- $R^2$ für Wasserstoff, Alkyl oder gegebenenfalls substituiertes Aryl steht,
- $R^3$ für Wasserstoff oder Alkyl steht,
- $R^4$ für Wasserstoff, Alkyl, Phenyl oder Alkylthio steht,
- $R^5$ für gegebenenfalls substituiertes Alkyl, gegebenenfalls substituiertes Cycloalkyl oder für den Rest

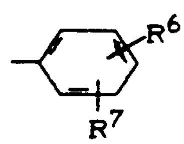

steht,

wobei $R^6$ und $R^7$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, Halogendialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl oder wobei $R^6$ und $R^7$ zusammen für einen bivalenten gegebenenfalls ein oder zwei Sauerstoffatome enthaltenden und gegebenenfalls substituierten Rest stehen,

- X für Sauerstoff oder Schwefel steht und
- Y und Z gleich oder verschieden sein können und für Wasserstoff, Alkyl, Halogen, Halogenalkyl, Alkoxy, Alkylthio, Halogenalkoxy, Halogenalkylthio, Alkylcarbonyl, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Amino, Nitro, Cyano oder wobei Y und Z zusammen für gegebenenfalls durch Halogen substituiertes 3,4-Methylendioxy oder 3,4-Ethylendioxy stehen,

dadurch gekennzeichnet, daß man

(a) zum Erhalt von Pyrazolinderivaten der Formel (Ia), in welcher $R^4$ für Wasserstoff steht,

(α) Pyrazolinderivate der Formel (II)

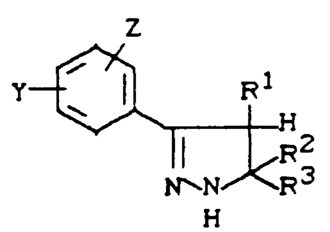

(II)

in welcher Y, Z, $R^1$, $R^2$ und $R^3$ die oben angegebene Bedeutung besitzen,

mit Isocyanaten bzw. Isothiocyanaten der Formel (III)

$X=C=N\text{-}R^5$ (III)

in welcher X und $R^5$ die oben angegebene Bedeutung haben,
gegebenenfalls in Gegenwart eines Verdünnungsmittels und gegebenenfalls in Gegenwart von Basen umsetzt

oder

($\beta$) substituierte Pyrazolcarbonsäureanilide der Formel (IV)

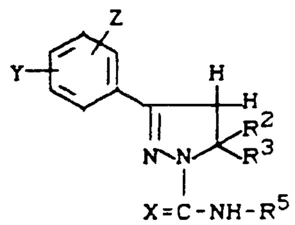

(IV)

in welcher

X, Y, Z, $R^2$, $R^3$ und $R^5$ die oben angegebene Bedeutung haben,

mit Halogeniden der Formel (V)

$Hal\text{-}R^1$ (V)

in welcher

$R^1$ die oben angegebene Bedeutung hat und

Hal für Fluor, Chlor, Brom, insbesondere Chlor oder Brom steht,

gegebenenfalls in Gegenwart eines Verdünnungsmittels und in Gegenwart einer starken Base und gegebenenfalls in Gegenwart einer Schutzgasatmosphäre umsetzt,

oder daß man

(b) zum Erhalt von Pyrazolinderivaten der Formel (Ib), in welcher $R^4$ für Alkyl, Phenyl oder Alkylthio steht, die nach Verfahren (a-$\alpha$) und (a-$\beta$) erhältlichen Pyrazolinderivate der Formel (Ia),

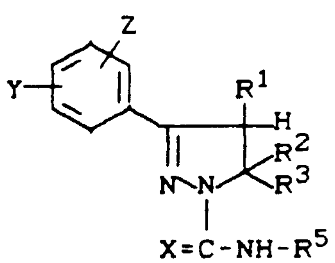

(Ia)

in welcher

X, Y, Z, $R^1$, $R^2$, $R^3$ und $R^5$ die oben angegebene Bedeutung haben,

mit Alkylierungsmitteln der Formel (VI)

$R^4\text{-}E$ (VI)

in welcher

$R^4$ für Alkyl, Phenyl oder Alkylthio steht und

E für eine elektronenanziehende Abgangsgruppe steht,

in wasserfreiem Medium unter Zusatz einer starken Base umsetzt.

**6.** 4-Hetaryl-pyrazolin-Derivate der allgemeinen Formel (II)

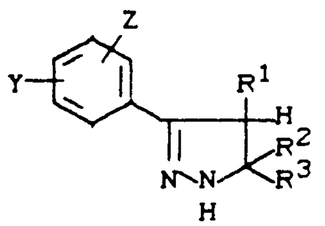

(II)

in welcher

$R^1$ für einen ungesättigten sechsgliedrigen, 1 bis 3 Stickstoffatome enthaltenden, gegebenenfalls substituierten und gegebenenfalls benzokondensierten Heterocyclus steht,

$R^2$ für Wasserstoff, Alkyl oder gegebenenfalls substituiertes Aryl steht,

$R^3$ für Wasserstoff oder Alkyl steht,

$R^4$ für Wasserstoff, Alkyl, Phenyl oder Alkylthio steht,

X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Amino, Nitro, Cyano oder wobei Y und Z zusammen für gegebenenfalls durch Halogen substituiertes 3,4-Methylendioxy oder 3,4-Ethylendioxy stehen.

**7.** Schädlingsbekämpfungsmittel, gekennzeichnet durch einen Gehalt an mindestens einem substituierten 4-Hetaryl-pyrazolin der Formel (I).

**8.** Verfahren zur Bekämpfung von tierischen Schädlingen, dadurch gekennzeichnet, daß man substituierte 4-Hetaryl-pyrazoline der Formel (I) auf tierische Schädlinge und/oder ihren Lebensraum einwirken läßt.

**9.** Verwendung von substituierten 4-Hetaryl-pyrazolinen der Formel (I) zur Bekämpfung von tierischen Schädlingen.

**10.** Verfahren zur Herstellung von Schädlingsbekämpfungsmitteln, dadurch gekennzeichnet, daß man substituierte 4-Hetaryl-pyrazoline der Formel (I) mit Streckmitteln und/oder oberflächenaktiven Mitteln vermischt.

**Patentansprüche für folgenden Vertragsstaat : ES**

**1.** Verfahren zur Herstellung von substituierten 4-Hetarylpyrazolinen der allgemeinen Formel (I)

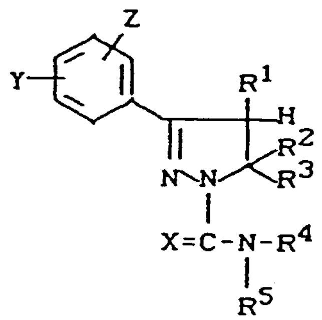

(I)

in welcher

$R^1$ für einen ungesättigten sechsgliedrigen, 1 bis 3 Stickstoffatome enthaltenden, gegebenenfalls substituierten und gegebenenfalls benzokondensierten Heterocyclus steht,

$R^2$ für Wasserstoff, Alkyl oder gegebenenfalls substituiertes Aryl steht,
$R^3$ für Wasserstoff oder Alkyl steht,
$R^4$ für Wasserstoff, Alkyl, Phenyl oder Alkylthio steht,
$R^5$ für gegebenenfalls substituiertes Alkyl, gegebenenfalls substituiertes Cycloalxyl oder für den Rest

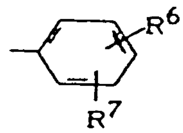

steht,

wobei $R^6$ und $R^7$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, Halogendialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl oder wobei $R^6$ und $R^7$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy,

Halogenalkythio, Alkylcarbonyl, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Amino, Nitro, Cyano oder wobei Y und Z zusammen für gegebenenfalls durch Halogen substituiertes 3,4-Methylendioxy oder 3,4-Ethylendioxy stehen,

dadurch gekennzeichnet, daß man

(a) zum Erhalt von Pyrazolinderivaten der Formel (Ia), in welcher $R^4$ für Wasserstoff steht,

(α) Pyrazolinderivate der Formel (II)

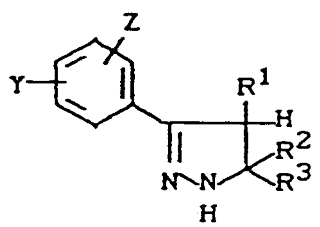

(II)

in welcher Y, Z, $R^1$, $R^2$ und $R^3$ die oben angegebene Bedeutung besitzen,

mit Isocyanaten bzw. Isothiocyanaten der Formel (III)

$X=C=N\text{-}R^5$ (III)

in welcher X und $R^5$ die oben angegebene Bedeutung haben,

gegebenenfalls in Gegenwart eines Verdünnungsmittels und gegebenenfalls in Gegenwart von Basen umsetzt

oder

(β) substituierte Pyrazolcarbonsäureanilide der Formel (IV)

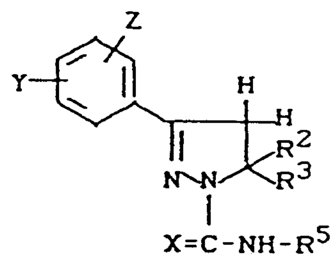

(IV)

in welcher

X, Y, Z, $R^2$, $R^3$ und $R^5$ die oben angegebene Bedeutung haben,

mit Halogeniden der Formel (V)

Hal-$R^1$ (V)

in welcher

$R^1$ die oben angegebene Bedeutung hat und

Hal für Fluor, Chlor, Brom, insbesondere Chlor oder Brom steht,

gegebenenfalls in Gegenwart eines Verdünnungsmittels und in Gegenwart einer starken Base und gegebenenfalls in Gegenwart einer Schutzgasatmosphäre umsetzt,

oder daß man

(b) zum Erhalt von Pyrazolinderivaten der Formel (Ib), in welcher $R^4$ für Alkyl, Phenyl oder Alkylthio steht, die nach Verfahren (a-$\alpha$) und (a-$\beta$) erhältlichen Pyrazolinderivate der Formel (Ia),

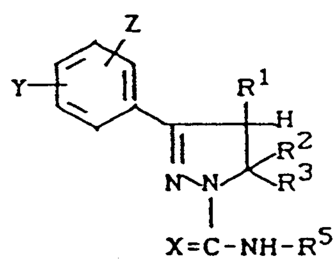

(Ia)

in welcher

X, Y, Z, $R^1$, $R^2$, $R^3$ und $R^5$ die oben angegebene Bedeutung haben,

mit Alkylierungsmitteln der Formel (VI)

$R^4$-E (VI)

in welcher

$R^4$ für Alkyl, Phenyl oder Alkylthio steht und

E für eine elektronenanziehende Abgangsgruppe steht,

in wasserfreiem Medium unter Zusatz einer starken Base umsetzt.

**2.** Verfahren gemäß Anspruch 1 zur Herstellung von substituierten 4-Hetaryl-pyrazolinen der allgemeinen Formel (I) gemäß Anspruch 1, in welcher

$R^1$ für einen gegebenenfalls durch Halogen, Hydroxy, Alkyl($C_1$-$C_6$), CN, $NO_2$, Alkoxy($C_1$-$C_6$)-carbonyl, Alkyl($C_1$-$C_4$)thio, Alkoxy($C_1$-$C_6$), Halogenalkyl($C_1$-$C_4$), Halogenalkyl($C_1$-$C_4$)thio, Halogenalkoxy($C_1$-$C_4$), Dialkyl-($C_1$-$C_4$)amino oder Dihalogenamino substituierten Pyridin-, Pyridon-, Pyrimidin-, Pyrimidon-, Pyridazinon-, Pyrazinon-, Pyrazin-, Pyridazin- oder Triazin-Rest steht,

$R^2$ für Wasserstoff, Alkyl($C_1$-$C_6$) oder gegebenenfalls einfach bis dreifach, gleich oder verschieden substituiertes Phenyl steht, wobei als Substituenten genannt seien: Halogen, Cyano, Alkyl($C_1$-$C_6$), Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio,

$R^3$ für Wasserstoff oder Alkyl($C_1$-$C_6$) steht,

$R^4$ für Wasserstoff, Alkyl($C_1$-$C_6$), Phenyl oder Alkyl($C_1$-$C_4$)thio steht,

$R^5$ für gegebenenfalls durch Halogen, Halogenalkyl($C_1$-$C_4$), Halogenalkoxy($C_1$-$C_4$) substituiertes Alkyl($C_1$-$C_6$),

für gegebenenfalls durch Halogen, Halogenalkyl($C_1$-$C_4$),Halogenalkoxy($C_1$-$C_4$) substituiertes Cycloalkyl ($C_3$-$C_7$) oder für den Rest

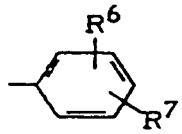

steht,

wobei $R^6$ und $R^7$ gleich oder verschieden sein können und für Wasserstoff, Halogen, Alkyl-($C_1$-$C_6$), Nitro, Cyano, Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio, gegebenenfalls durch Halogen, Halogenalkyl($C_1$-$C_6$), Alkoxy-($C_1$-$C_6$), Alkyl($C_1$-$C_6$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_6$)carbonyl, Alkenyl($C_2$-$C_6$)oxy, Alkinyl($C_2$-$C_6$), Alkyl($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)-carbonyl, Alkyl($C_1$-$C_4$)-carbonyl, stehen oder wobei

$R^6$ und $R^7$ zusammen für einen der folgenden bivalenten Reste stehen:

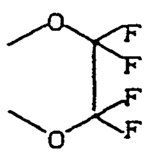 , 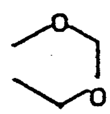 , 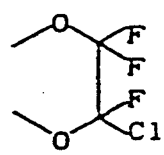 , 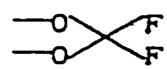 ,

X für Sauerstoff oder Schwefel steht und

Y und Z gleich oder verschieden sein können und für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_4$), Halogenalkyl($C_1$-$C_4$)thio, Alkyl($C_1$-$C_4$)carbonyl, Alkoxy($C_1$-$C_4$)carbonyl, Halogenalkoxy($C_1$-$C_4$)-carbonyl, gegebenenfalls durch Halogen, Alkyl($C_1$-$C_4$), Alkoxy($C_1$-$C_4$), Halogenalkyl ($C_1$-$C_4$) substituiertes Phenoxy oder Phenylthio, Alkenyl($C_2$-$C_6$)oxy, Alkinyl($C_2$-$C_6$), Alkyl($C_1$-$C_4$)-thionyl, Alkyl($C_1$-$C_4$)sulfonyl, Halogenalkyl($C_1$-$C_4$)thionyl, Halogenalkyl($C_1$-$C_4$)sulfonyl, gegebenenfalls durch Halogen, Alkoxy($C_1$-$C_4$), Halogenalkyl($C_1$-$C_4$) substituiertes Mono- oder Dialkyl($C_1$-$C_6$)amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

**3.** Verfahren gemäß Anspruch 1 zur Herstellung von substituierten 4-Hetaryl-pyrazolinen der allgemeinen Formel (I) gemäß Anspruch 1, in welcher

$R^1$ für einen gegebenenfalls durch Fluor, Chlor, Brom, Iod, Hydroxy, Alkyl($C_1$-$C_4$), CN, $NO_2$, Alkoxy($C_1$-$C_4$)carbonyl, Alkyl($C_1$-$C_3$)thio, Alkoxy($C_1$-$C_4$), Halogenalkyl($C_1$-$C_3$), Halogenalkyl-($C_1$-$C_3$)thio oder Halogenalkoxy($C_1$-$C_4$)-carbonyl substituierten Pyridin-, Pyridon-, Pyrimidin-, Pyrimidon-, Pyridazinon-, Pyrazinon-, Pyrazin-, Pyridazin- oder Triazin-Rest steht,

$R^2$ für Wasserstoff, Alkyl($C_1$-$C_4$) oder gegebenenfalls durch Halogen, Cyano, Alkyl($C_1$-$C_3$), Halogenalkyl($C_1$-$C_3$), Alkoxy($C_1$-$C_3$), Halogenalkoxy($C_1$-$C_3$), Alkyl($C_1$-$C_3$)thio oder Halogenalkyl($C_1$-$C_3$)thio substituiertes Phenyl steht,

$R^3$ für Wasserstoff oder Alkyl($C_1$-$C_4$) steht,

$R^4$ für Wasserstoff, Alkyl($C_1$-$C_4$), Phenyl oder Alkyl($C_1$-$C_3$)thio steht,

$R^5$ für gegebenenfalls durch Fluor, Chlor, Brom, Halogenalkyl($C_1$-$C_3$), Halogenalkoxy($C_1$-$C_3$)-substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cycloalkyl($C_3$-$C_6$) oder für den Rest

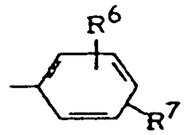

steht,

wobei $R^6$ und $R^7$ gleich oder verschieden sein können und für

Wasserstoff, Fluor, Chlor, Brom, Iod, Alkyl($C_1$-$C_4$), Nitro, Cyano, Halogenalkyl($C_1$-$C_3$), Alkoxy-($C_1$-$C_4$), Halogenalkoxy ($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$), Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituierts Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$),Alkoxy($C_1$-$C_3$)carbonyl, Alkyl($C_1$-$C_3$)thionyl, Alkyl($C_1$-$C_3$)carbonyl, Halogenalkyl ($C_1$-$C_3$)sulfuryl stehen oder wobei

$R^6$ und $R^7$ zusammen für einen der folgenden bivalenten Reste stehen:

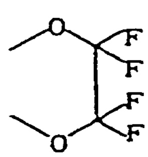 , 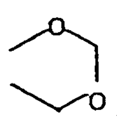 ; 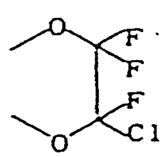 , 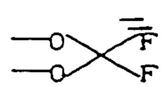 ,

X für Sauerstoff oder Schwefel steht und

Y und Z gleich oder verschieden sein können und für Wasserstoff, Alkyl($C_1$-$C_4$), Fluor, Chlor, Brom, Jod, Halogenalkyl($C_1$-$C_4$), Alkoxy($C_1$-$C_4$), Alkyl($C_1$-$C_4$)thio , Halogenalkoxy($C_1$-$C_3$), Halogenalkyl($C_1$-$C_3$)thio,

Alkoxy($C_1$-$C_3$)carbonyl, gegebenenfalls durch Fluor, Chlor, Brom, Alkyl($C_1$-$C_3$), Alkoxy($C_1$-$C_3$), Halogenalkyl($C_1$-$C_3$) substituiertes Phenoxy oder Phenylthio, Halogenalkoxy($C_1$-$C_3$)carbonyl, Alkenyl($C_2$-$C_4$)oxy, Alkinyl($C_2$-$C_4$), Alkyl($C_1$-$C_3$) thionyl, Alkyl($C_1$-$C_3$)-sulfonyl, Halogenalkyl-($C_1$-$C_3$) thionyl, Halogenalkyl($C_1$-$C_3$)sulfonyl, gegebenenfalls durch Halogen, Alkoxy ($C_1$-$C_3$), Halogenalkyl ($C_1$-$C_3$), substituiertes Mono- oder Dialkyl ($C_1$-$C_3$)-amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

**4.** Schädlingsbekämpfungsmittel, gekennzeichnet durch einen Gehalt an mindestens einem substituierten 4-Hetaryl-pyrazolin der Formel (I).

**5.** Verfahren zur Bekämpfung von tierischen Schädlingen, dadurch gekennzeichnet, daß man substituierte 4-Hetaryl-pyrazoline der Formel (I) auf tierische Schädlinge und/oder ihren Lebensraum einwirken läßt.

**6.** Verwendung von substituierten 4-Hetaryl-pyrazolinen der Formel (I) zur Bekämpfung von tierischen

Schädlingen.

7. Verfahren zur Herstellung von Schädlingsbekämpfungsmitteln, dadurch gekennzeichnet, daß man substituierte 4-Hetaryl-pyrazoline der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 92 10 7950

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | US-A-4 070 365 (U.S. PHILIPS CORPORATION)<br>--- | 1-10 | C07D401/04<br>A01N43/56<br>C07D403/04<br>C07D405/14 |
| A | FR-A-2 171 218 (N.V. PHILIPS' GLOEILAMPENFABRIEKEN)<br>--- | | |
| A | NL-A-7 301 203 (N.V. PHILIPS' GLOEILAMPENFABRIEKEN)<br>--- | | |
| D,A | US-A-4 174 393 (DUPHAR INTERNATIONAL RESEARCH B.V.)<br>--- | | |
| P,A | EP-A-0 438 690 (BAYER AKTIENGESELLSCHAFT)<br>----- | | |
| | | | RECHERCHIERTE SACHGEBIETE (Int. Cl.5) |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 19 AUGUST 1992 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)